# EUROPEAN PATENT SPECIFICATION

(11) **EP 1 281 709 B1**
(45) Date of publication and mention of the grant of the patent: **16.02.2005**
(21) Application number: 01921386.7
(22) Date of filing: 23.04.2001
(51) Int. Cl.: C07D 403/10, C07D 403/12, C07D 403/14, C07D 405/10, C07D 405/12, C07D 405/14, C07D 413/10, C07D 413/12, C07D 413/14, A61K 31/4155, A61K 31/4178, A61K 31/341, A61P 29/00, A61P 35/00

(54) **NOVEL HETEROCYCLIC COMPOUNDS WITH ANTI-INFLAMMATORY ACTIVITY**
HETEROZYKLISCHE VERBINDUNGEN MIT ENTZÜNDUNGSHEMMENDER WIRKUNG
NOUVEAUX COMPOSES HETEROCYCLIQUES A ACTIVITE ANTI-INFLAMMATOIRE

(30) Priority: 25.04.2000 ES 200001138
(43) Date of publication of application: 05.02.2003
(73) Proprietor: J. Uriach y Compania S.A., 08184 Palau-solità i Plegamans (Barcelona) (ES)
(72) Inventor: ALMANSA ROSALES, Carmen, E-08026 Barcelona (ES); GONZALEZ GONZALEZ, Concepción, E-08830 Sant Boi de Llobregat (ES); TORRES BARREDA, M. Carmen, E-08912 Badalona (ES)
(74) Representative: Zumstein, Fritz, Dr.
(86) International application number: PCT/ES2001/000152
(87) International publication number: WO 2001/083475

(56) References cited:
- WO-A-00/23426
- WO-A-00/66562
- WO-A-01/16138
- WO-A-01/17996
- WO-A-94/26731
- WO-A-95/00501
- WO-A-97/27181
- WO-A-97/38986
- WO-A-99/14205
- WO-A-99/64415
- P. PRASIT ET AL.: 'Selective cyclooxygenase-2 inhibitors' ANNUAL REPORTS IN MEDICINAL CHEMISTRY vol. 32, 1997, pages 211 - 220, XP001038176
- J. TALLY ET AL.: 'Discovery of selective COX-2 inhibitors' ACTUALITES DE CHIMIE THERAPEUTIQUE vol. 25, 1999, pages 123 - 134, XP001026553
- I. KHANNA ET AL.: '1,2-Diarylimidazoles as potent, cyclooxygenase-2 selective and orally active antiinflammatory agents' JOURNAL MEDICINAL CHEMISTRY vol. 40, 1997, pages 1634 - 1647, XP000941303

## Description

### Field of the invention.

The present invention relates to a new series of heterocyclic compounds with anti-inflammatory activity, as well as to a process for their preparation, to the pharmaceutical compositions containing them and to their use in medicine.

### Description of the arior art.

In many acute as well as chronic inflammatory processes, substances derived from the metabolism of arachidonic acid are involved. These substances form a large family of compounds of lipidic nature that are the result of the action of a series of enzymes which form what is called the arachidonic acid cascade. The most important one from the therapeutic point of view is prostaglandin G/H synthase (PGHS), also known as cyclooxygenase (COX), which catalyzes the formation of vasoactive and inflammatory substances such as prostaglandins (PGE₂, PGD₂, PGF₂), prostacyclin (PGl₂) and thromboxane A₂ (TXA₂).

Inhibition of cyclooxygenase (COX) is the mechanism of action responsible for the effect of most anti-inflammatory drugs on the market (non-steroidal anti-inflammatory drugs, NSAIDs). Said inhibition also reduces the levels of prostaglandins at gastric level, which has been correlated to the well known gastric effects of NSAIDs in view of the protective role of said molecules on the gastric mucosa.

In the early 90's two cyclooxygenase isoforms, COX-1 and COX-2, were described. COX-1 is the constitutive isoform, present in many tissues, but preferentially in the stomach, kidney and platelets. Its inhibition is responsible for the gastric and renal effects of NSAIDs. On the other hand, COX-2 is an inducible isoform, which is expressed as a consequence of an inflammatory or mitogenic stimulus in a wide range of tissues such as macrophages, chondrocytes, fibroblasts and endothelial cells.

The discovery of the inducible isoenzyme of PGHS (PGHS₂ or COX-2) has allowed the synthesis of selective COX-2 inhibitors which presumably improve the gastric tolerance of these drugs, since as they inhibit the constitutive form present in the stomach to a lesser extent, they exhibit reduced ulcerogenic potency (one of the most characteristic side effects of non-selective inhibitors). The present invention describes new cyclooxygenase inhibitors with selectivity for the isoform 2 (COX-2).

### Description of the invention.

The present invention relates to the new compounds of general formula **I**: 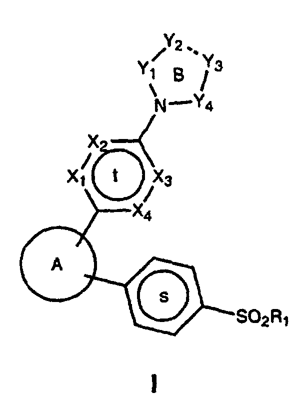 wherein:
A represents an insaturated or partially insaturated 5-membered ring, which can optionally contain from 1 to 3 heteroatoms selected from N, O and S, wherein the two aromatic substituents s and t are placed on adjacent atoms of ring A, and
wherein A can be optionally substituted with one or more substituents R₂;
R₁ represents C₁₋₈ alkyl, C₁₋₈ haloalkyl or -NR₃R₄;
R₂ represents C₁₋₄ alkyl, C₁₋₄ haloalkyl, halogen, oxo, cyano, nitro, -CHO, -COCH₃ or -COOR₃;
R₃ represents hydrogen, C₁₋₈ alkyl or arylC₀₋₈ alkyl;
R₄ represents hydrogen, C₁₋₈ alkyl, arylC₁₋₈ alkyl, -COR₅ or -COOR₅;
R₅ represents C₁₋₈ alkyl or C₁₋₈ haloalkyl;
X₁, X₂, X₃ and X₄ represent all CR₆, or one, two or three of X₁, X₂, X₃ and X₄ represent nitrogen and the other groups X₁, X₂, X₃ and X₄ represent CR₆;
each R₆ independently represents hydrogen, halogen, C₁₋₃ alkyl or C₁₋₃ alkoxy;
the dotted line in ring B represents a single or double bond;
Y₁ and Y₄ independently represent CR₇R₇ or CO;
when the dotted line represents a double bond Y₂ and Y₃ represent CR₈;
when the dotted line represents a single bond Y₂ and Y₃ represent CR₈R₈, and additionally Y₂ can represent CO if Y₁ is different from CO, and additionally Y₃ can represent CO if Y₄ is different from CO or Y₃ can represent NR₉, O or S if Y₄ represents CO;
each R₇ independently represents hydrogen, methyl or ethyl;
each R₈ independently represents hydrogen, methyl, ethyl, hydroxy, methoxy or halogen;
R₉ represents hydrogen or C₁₋₄ alkyl;
aryl in the above definitions represents phenyl or naphthyl, which can be optionally substituted with one or more groups selected from C₁₋₈ alkyl, halogen, C₁₋₈ haloalkyl, cyano, nitro, R₁₀OC₀₋₈ alkyl, R₁₀SC₀₋₈ alkyl, -NR₁₀R₁₁, -NA₁₀COR₁₁, -COR₁₀ or -COOR₁₀;
R₁₀ represents hydrogen, C₁₋₈ alkyl or benzyl;
R₁₁ represents C₁₋₈ alkyl or C₁₋₈ haloalkyl.

The present invention also relates to the addition salts of the compounds of the invention as well as to their solvates.

Some compounds of formula **I** can have chiral centres, which can give rise to various stereoisomers. The present invention relates to each one of the individual stereoisomers as well as to their mixtures.

The present invention also relates to the pharmaceutical compositions which comprise an effective amount of a compound of formula **I** or a pharmaceutically acceptable salt, or solvate thereof and one or more pharmaceutically acceptable excipients.

The present invention also relates to the use of a compound of formula **I** or a pharmaceutically acceptable salt, or solvate thereof for the manufacture of a medicament for the treatment or prevention of diseases mediated by cyclooxygenase, especially cyclooxygenase-2.

The present invention also relates to the use of a compound of formula **I** or a pharmaceutically acceptable salt, or solvate thereof for the manufacture of a medicament for the treatment of inflammation, pain and/or fever.

The present invention also relates to the use of a compound of formula **I** or a pharmaceutically acceptable salt, or solvate thereof for the manufacture of a medicament for inhibiting prostanoid-induced smooth muscle contraction.

The present invention also relates to the use of a compound of formula **I** or a pharmaceutically acceptable salt, or solvate thereof for the manufacture of a medicament for the treatment or prevention of dysmenorrhea, preterm labour, asthma and bronchitis.

The present invention also relates to the use of a compound of formula or a pharmaceutically acceptable salt, or solvate thereof for the manufacture of a medicament for the treatment or prevention of familial adenomatous polyposis.

The present invention also relates to the use of a compound of formula **I** or a pharmaceutically acceptable salt, or solvate thereof for the manufacture of a medicament for the treatment or prevention of cancer, preferably gastrointestinal cancers, and more preferably colon cancer.

The present invention also relates to the use of a compound of formula **I** or a pharmaceutically acceptable salt, or solvate thereof for the manufacture of a medicament for the treatment or prevention of cerebral infarction, epilepsy, and neurodegenerative diseases such as Alzheimer's disease and dementia.

The present invention also relates to a compound of formula **I** or a pharmaceutically acceptable salt, or solvate thereof for the treatment or prevention of diseases mediated by cyclooxygenase, especially cyclooxygenase-2.

The present invention also relates to a compound of formula **I** or a pharmaceutically acceptable salt, or solvate thereof for the treatment of inflammation, pain and/or fever.

The present invention also relates to a compound of formula **I** or a pharmaceutically acceptable salt, or solvate thereof for inhibiting prostanoid-induced smooth muscle contraction.

The present invention also relates to a compound of formula **I** or a pharmaceutically acceptable salt, or solvate thereof for the treatment or prevention of dysmenorrhea, preterm labour, asthma and bronchitis.

The present invention also relates to a compound of formula **I** or a pharmaceutically acceptable salt, or solvate thereof for the treatment or prevention of familial adenomatous polyposis.

The present invention also relates to a compound of formula **I** or a pharmaceutically acceptable salt, or solvate thereof for the treatment or prevention of cancer, preferably gastrointestinal cancers, and more preferably colon cancer.

The present invention also relates to a compound of formula **I** or a pharmaceutically acceptable salt, or solvate thereof for the treatment or prevention of cerebral infarction, epilepsy, and neurodegenerative diseases such as Alzheimer's disease and dementia.

The present invention also relates to the use of a compound of formula **I** or a pharmaceutically acceptable salt, or solvate thereof for the treatment or prevention of diseases mediated by cyclooxygenase, especially cyclooxygenase-2.

The present invention also relates to the use of a compound of formula **I** or a pharmaceutically acceptable salt, or solvate thereof for the treatment of inflammation, pain and/or fever.

The present invention also relates to the use of a compound of formula **I** or a pharmaceutically acceptable salt, or solvate thereof for inhibiting prostanoid-induced smooth muscle contraction.

The present invention also relates to the use of a compound of formula **I** or a pharmaceutically acceptable salt, or solvate thereof for the treatment or prevention of dysmenorrhea, preterm labour, asthma and bronchitis.

The present invention also relates to the use of a compound of formula **I** or a pharmaceutically acceptable salt, or solvate thereof for the treatment or prevention of familial adenomatous polyposis.

The present invention also relates to the use of a compound of formula **I** or a pharmaceutically acceptable salt, or solvate thereof for the treatment or prevention of cancer, preferably gastrointestinal cancers, and more preferably colon cancer.

The present invention also relates to the use of a compound of formula **I** or a pharmaceutically acceptable salt, or solvate thereof for the treatment or prevention of cerebral infarction, epilepsy, and neurodegenerative diseases such as Alzheimer's disease and dementia.

The present invention also relates to a method for the treatment or prevention of diseases mediated by cyclooxygenase, especially cyclooxygenase-2, in a mammal in need thereof, especially a human being, which comprises administering to said mammal a therapeutically effective amount of a compound of formula **I** or a pharmaceutically acceptable salt, or solvate thereof.

The present invention also relates to a method for the treatment of inflammation, pain and/or fever in a mammal in need thereof, especially a human being, which comprises administering to said mammal a therapeutically effective amount of a compound of formula **I** or a pharmaceutically acceptable salt, or solvate thereof.

The present invention also relates to a method of inhibiting prostanoid-induced smooth muscle contraction in a mammal in need thereof, especially a human being, which comprises administering to said mammal a therapeutically effective amount of a compound of formula **I** or a pharmaceutically acceptable salt, or solvate thereof.

The present invention also relates to a method for the treatment or prevention of dysmenorrhea, preterm labour, asthma and bronchitis in a mammal in need thereof, especially a human being, which comprises administering to said mammal a therapeutically effective amount of a compound of formula **I** or a pharmaceutically acceptable salt, or solvate thereof.

The present invention also relates to a method for the treatment or prevention of familial adenomatous polyposis in a mammal in need thereof, especially a human being, which comprises administering to said mammal a therapeutically effective amount of a compound of formula **I** or a pharmaceutically acceptable salt, or solvate thereof.

The present invention also relates to a method for the treatment or prevention of cancer, preferably gastrointestinal cancers, and more preferably colon cancer, in a mammal in need thereof, especially a human being, which comprises administering to said mammal a therapeutically effective amount of a compound of formula **I** or a pharmaceutically acceptable salt, or solvate thereof.

The present invention also relates to a method for the treatment or prevention of cerebral infarction, epilepsy, and neurodegenerative diseases such as Alzheimer's disease and dementia in a mammal in need thereof, especially a human being, which comprises administering to said mammal a therapeutically effective amount of a compound of formula **I** or a pharmaceutically acceptable salt, or solvate thereof.

Another object of the present invention is to provide a process for preparing a compound of formula **I**, which comprises:
(a) when in a compound of formula **I** Y₁ and Y₄ represent CR₇R₇, reacting a compound of formula **II** 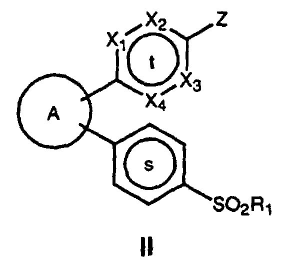 wherein X₁, X₂, X₃, X₄, R₁ and A have the meaning described above and Z represents fluoro or, when one of X₂ or X₃ represents nitrogen, Z represents chloro, with an amine of formula **IV** 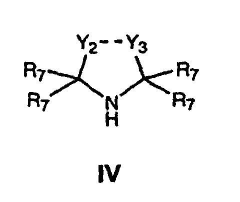 wherein R₇, Y₂, Y₃ and the dotted line have the meaning described above; or
(b) when in a compound of formula **I** Y₁ and Y₄ represent CR₇R₇, reacting a compound of formula **III** 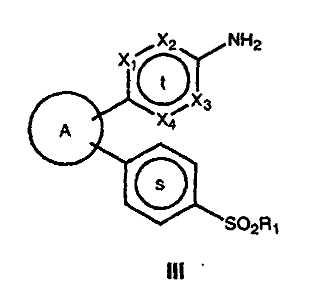 wherein X₁, X₂, X₃, X₄, R₁ and A have the meaning described above, with a compound of formula **V** 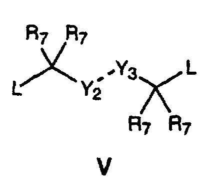 wherein R₇, Y₂, Y₃ and the dotted line have the meaning described above and L represents a good leaving group; or
(c) when in a compound of formula **I** Y₁ and/or Y₄ represents CO, reacting a compound of formula **III**, defined above, with a compound of formula **VI** or **VI'** respectively 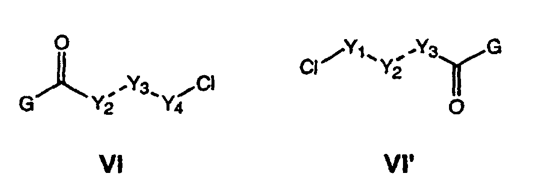 wherein Y₁, Y₂, Y₃, Y₄ and the dotted line have the meaning described above and G represents a good leaving group; or
(d) when in a compound of formula **I** Y₄ represents CO and Y₃ represents NH, reacting a compound of formula **III**, defined above, with an isocyanate of formula **VII** 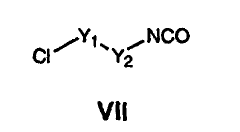 wherein Y₁ and Y₂ have the meaning described above; or
(e) when in a compound of formula **I** Y₁ and Y₄ represent CO, reacting a compound of formula **III**, defined above, with a compound of formula **VIII** 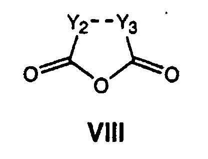 wherein Y₂, Y₃ and the dotted line have the meaning described above; or
(f) when in a compound of formula **I** one of Y₁ or Y₄ represents CH₂ and the other represents CO, and the dotted line represents a double bond, reacting a compound of formula **III**, defined above, with a compound of formula **IX** 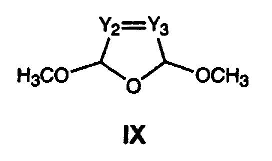 wherein Y₂ and Y₃ have the meaning described above; or
(g) converting, in one or a plurality of steps, a compound of formula **I** into another compound of formula **I**; and
(h) if desired, after the above steps, reacting a compound of formula **I** with an acid or a base to give the corresponding salt.

In the above definitions, the term C₁₋ₙ alkyl, as a group or part of a group, means a lineal or branched alkyl group containing from 1 to n carbon atoms. Thus, for example, when n is 8 it includes among others the groups methyl, ethyl, propyl, isopropyl, butyl, isobutyl, *sec*-butyl, *tert*-butyl, pentyl, isopentyl, neopentyl, hexyl, heptyl and octyl. A group C₀₋₈ alkyl means that additionally the alkyl group can be absent when it is C₀ alkyl (i.e., that a covalent bond is present).

A halogen radical or its abbreviation halo means fluoro, chloro, bromo or iodo.

A C₁₋ₙ haloalkyl group means a group resulting from the substitution of one or more hydrogen atoms of a C₁₋ₙ alkyl group with one or more halogen atoms (that is, fluoro, chloro, bromo or iodo), which can be the same or different. When n is 8 it includes among others trifluoromethyl, fluoromethyl, 1-chloroethyl, 2-chloroethyl, 1-fluoroethyl, 2-fluoroethyl, 2-bromoethyl, 2-iodoethyl, 2,2,2-trifluoroethyl, pentafluoroethyl, 3-fluoropropyl, 3-chloropropyl, 2,2,3,3-tetrafluoropropyl, 2,2,3,3,3-pentafluoropropyl, heptafluoropropyl, 4-fluorobutyl, nonafluorobutyl, 5-fluoropentyl, 6-fluorohexyl, 7-fluoroheptyl and 8-fluorooctyl.

A C₁₋₃ alkoxy group means a group resulting from the union of a C₁₋₃ alkyl group to an ether-type oxygen atom and it includes the methoxy, ethoxy, propoxy and isopropoxy groups.

An oxo group represents a carbonyl group. In the same way, CO represents a carbonyl group.

An arylC₁₋ₙ alkyl group means a group resulting from the substitution of an hydrogen atom of a C₁₋ₙ alkyl group with an aryl group as defined above, that is phenyl or naphthyl, which can be optionally substituted as described above. Thus, for example, when n is 8 it includes among others benzyl, 1-phenylethyl, 2-phenylethyl, 3-phenylpropyl, 2-phenylpropyl, 1-phenylpropyl, 4-phenylbutyl, 3-phenylbutyl, 2-phenylbutyl, 1-phenylbutyl, 5-phenylpentyl, 6-phenylhexyl, 7-phenylheptyl and 8-phenyloctyl, wherein the phenyl group can be optionally substituted. An arylC₀₋ₙ alkyl group means that it additionally includes an aryl group when the alkyl group is absent (that is, when it is C₀ alkyl).

In the compounds of the present invention, A represents an insaturated or partially insaturated 5-membered ring which can be carbocyclic or heterocyclic, in which case it can contain from 1 to 3 heteroatoms selected from N, O and S. The two aromatic substituents s and t, i.e. the 4-(R₁SO₂)-phenyl group and the aromatic group substituted at the para position with ring B, are placed on adjacent ring atoms in ring A. This ring A can be unsubstituted or can additionally have one or more, preferably from one to three, substituents R₂, defined above, which can be the same or different and can be placed at any available position of ring A. As preferred examples of ring A, imidazole, pyrazole, furanone, thiophene, isoxazole, oxazole, oxazolone and cyclopentene can be mentioned.

Although the present invention includes all the compounds above mentioned, the preferred compounds of formula **I** are those wherein, independently or in any compatible combination:
A represents imidazole, pyrazole, furanone, thiophene, isoxazole, oxazole, oxazolone or cyclopentene, preferably imidazole, furanone or pyrazole, wherein A can be optionally substituted with one or more substituents R₂; and/or
R₁ represents methyl or amino; and/or
X₁, X₂, X₃ and X₄ represent all CR₆ or one of X₂ or X₃ represents N and the others represent CR₆; and/or
R₇ and R₈ represent hydrogen; and/or
Y₁, Y₂, Y₃ and Y₄ represent CH₂ and the dotted line represents a single bond, or one of Y₁ or Y₄ represents CO and the other represents CH₂ and Y₂ and Y₃ represent CH₂ in which case the dotted line represents a single bond or Y₂ and Y₃ represent CH in which case the dotted line represents a double bond.

Thus, a preferred class of compounds of the present invention are those compounds of formula **I** wherein R₁ represents methyl or amino.

Another preferred class of compounds of the present invention are those compounds of formula **I** wherein X₁, X₂, X₃ and X₄ represent all CR₆ or one of X₂ or X₃ represents N and the others represent CR₆.

Another preferred class of compounds of the present invention are those compounds of formula **I** wherein A represents imidazole, pyrazole, furanone, thiophene, isoxazole, oxazole, oxazolone or cyclopentene, wherein A can be optionally substituted with one or more substituents R₂.

A more preferred class of compounds of the present invention are those compounds of formula **I** wherein:
A represents imidazole, pyrazole, furanone, thiophene, isoxazole, oxazole, oxazolone or cyclopentene, wherein A can be optionally substituted with one or more substituents R₂;
R₁ represents methyl or amino; and
X₁, X₂, X₃ and X₄ represent all CR₆ or one of X₂ or X₃ represents N and the others represent CR₆.

A still more preferred class of compounds of the present invention are those compounds of formula **I** wherein:
A represents imidazole, pyrazole, furanone, thiophene, isoxazole, oxazole, oxazolone or cyclopentene, wherein A can be optionally substituted with one or more substituents R₂;
R₁ represents methyl or amino;
X₁, X₂, X₃ and X₄ represent all CR₆ or one of X₂ or X₃ represents N and the others represent CR₆; and
R₇ and R₈ represent hydrogen.

A particularly preferred class of compounds of the present invention are those compounds of formula **I** wherein:
A represents imidazole, pyrazole, furanone, thiophene, isoxazole, oxazole, oxazolone or cyclopentene, wherein A can be optionally substituted with one or more substituents R₂;
R₁ represents methyl or amino;
X₁, X₂, X₃ and X₄ represent all CR₆ or one of X₂ or X₃ represents N and the others represent CR₆; and
Y₁, Y₂, Y₃ and Y₄ represent CH₂ and the dotted line represents a single bond, or one of Y₁ or Y₄ represents CO and the other represents CH₂ and Y₂ and Y₃ represent CH₂ in which case the dotted line represents a single bond or Y₂ and Y₃ represent CH in which case the dotted line represents a double bond.

A still more particularly preferred class of compounds of the present invention are those compounds of formula **I** wherein:
A represents imidazole, furanone or pyrazole, wherein A can be optionally substituted with one or more substituents R₂;
R₁ represents methyl or amino;
X₁, X₂, X₃ and X₄ represent all CR₆ or one of X₂ or X₃ represents N and the others represent CR₆; and
Y₁, Y₂, Y₃ and Y₄ represent CH₂ and the dotted line represents a single bond, or one of Y₁ or Y₄ represents CO and the other represents CH₂ and Y₂ and Y₃ represent CH₂ in which case the dotted line represents a single bond or Y₂ and Y₃ represent CH in which case the dotted line represents a double bond.

The compounds of the present invention contain one or more basic nitrogens and, consequently, they can form salts with organic as well as inorganic acids, which are also included in the present invention. Examples of said salts include salts with inorganic acids such as hydrochloric acid, hydrobromic acid, hydroiodic acid, nitric acid, perchloric acid, sulfuric acid or phosphoric acid; and salts with organic acids, such as methanesulfonic acid, trifluoromethanesulfonic acid, ethanesulfonic acid, benzenesulfonic acid, *p*-toluenesulfonic acid, fumaric acid, oxalic acid, acetic acid or maleic acid, among others. The compounds of formula **I** where R¹ = -NHR₄ can also form salts with bases, which are also included in the present invention; examples thereof include salts with inorganic cations such as sodium, potassium, calcium, magnesium, lithium, aluminum, zinc, etc. There is no limitation on the nature of these salts, provided that when used for therapeutic purposes they are pharmaceutically acceptable. The salts can be prepared by treatment of a compound of formula **I** with a sufficient amount of the desired acid or base to give the salt in a conventional manner. The compounds of formula **I** and their salts differ in certain physical properties, such as solubility, but they are equivalent for the purposes of the invention.

Some compounds of the present invention can exist in solvated form, including hydrated forms. In general, the solvated forms, with pharmaceutically acceptable solvents such as water, ethanol and the like, are equivalent to the unsolvated form for the purposes of the invention.

Some compounds of the present invention can exist as various diastereoisomers and/or optical isomers. Diastereoisomers can be separated by conventional techniques such as chromatography or fractional crystallization. The optical isomers can be -resolved using conventional techniques of optical resolution, to give the optically pure isomers. This resolution can be performed upon any chiral synthetic intermediate or upon the products of general formula **I**. The optically pure isomers can also be individually obtained using enantiospecific synthesis. The present invention covers both the individual isomers and the mixtures (for example racemic mixtures), whether obtained by synthesis or by physically mixing them up.

It is also an object of the present invention to provide a process for preparing the compounds of formula **I**. As it will be obvious to a person skilled in the art, the precise method used for the preparation of a given compound can vary depending on its chemical structure. Furthermore, in most of the processes that are detailed below it may be necessary or appropriate to protect the reactive or labile groups using conventional protecting groups. Both the nature of said protecting groups and the processes for their introduction and removal are well known and belong to the state of the art (see for example Greene T.W. and Wuts P.G.M., "Protective Groups in Organic Synthesis", 3^{rd} Edition, John Wiley & Sons, 1999).

In general, the compounds of formula **I** can be prepared either by introducing ring B onto a precursor compound containing a halogen atom such as fluoro or chloro instead of ring B or by building up ring B starting from a precursor compound containing an amino group instead of ring B.

The compounds of formula **I** wherein Y₁ and Y₄ represent CR₇R₇ are in general obtained by reacting a halogenated compound of formula **II** with a cyclic amine of formula **IV,** as shown in the following scheme: 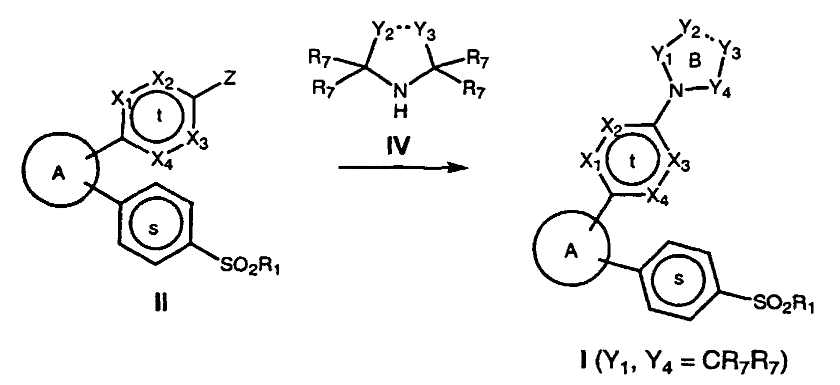 wherein Y₁ and Y₄ represent CR₇R₇, Z represents fluoro or, when one of X₂ or X₃ represents nitrogen, Z represents chloro, and R₁, R₇, X₁, X₂, X₃, X₄, A, Y₂, Y₃ and the dotted line have the meaning described above.

The reaction is carried out by heating the mixture of compounds of formulae **II** and **IV,** preferably at a temperature of about 150 °C, optionally in the presence of a suitable solvent such as tetrahydrofuran and optionally under pressure.

Alternatively, the compounds of formula **I** wherein Y₁ and Y₄ represent CR₇R₇ can also be obtained by reacting an amine of formula **III** with a compound of formula **V,** as shown in the following scheme: 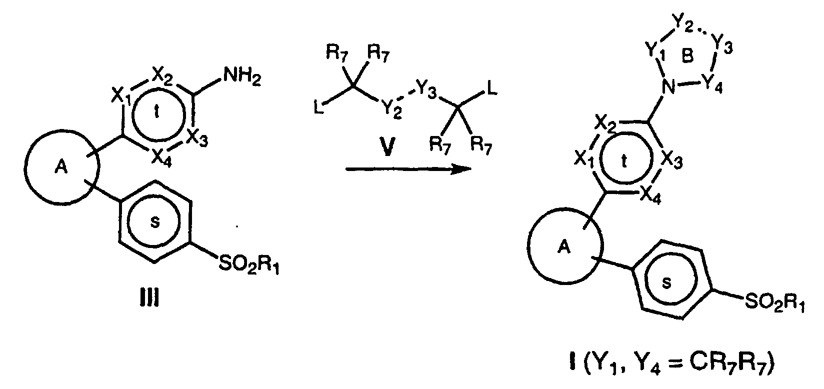 wherein Y₁ and Y₄ represent CR₇R₇, R₁, R₇, X₁, X₂, X₃, X₄, A, Y₂, Y₃ and the dotted line have the meaning described above and L represents a good leaving group, such as a halogen atom.

The reaction is carried out in the presence of a base such as triethylamine, in a suitable solvent such as acetonitrile, and heating, preferably at reflux.

The compounds of formula I wherein Y₁ and/or Y₄ represent CO can in general be obtained by reacting a compound of formula **III** with a compound of formula **VI** or **VI'** respectively 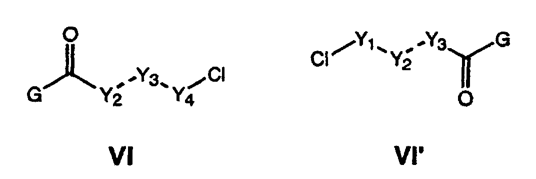 wherein Y₁, Y₂, Y₃, Y₄ and the dotted line have the meaning described above and G is a good leaving group such as chloro or phenoxy. Examples of compounds **VI** or **VI'** include, among others, 2-chloroethyl chloroformate, 4-chlorobutyryl chloride, butanedioyl dichloride and 2-butenedioyl dichloride.

The reaction is carried out in the presence of a proton scavenger amine such as triethylamine or pyridine, in a suitable solvent such as tetrahydrofuran or dimethylformamide, preferably at room temperature. Ring formation can be promoted by the addition of a base such as potassium *tert*-butoxide or potassium hydroxide.

The compounds of formula **I** wherein Y₄ represents CO and Y₃ represents NH can be obtained by reacting a compound of formula **III** with an isocyanate of formula **VII** 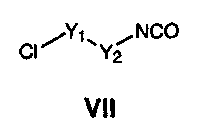 wherein Y₁ and Y₂ have the meaning described above.

The reaction is carried out in a suitable solvent such as tetrahydrofuran, dimethylformamide or chloroform at a temperature comprised between room temperature and that of the boiling point of the solvent. Ring formation can be promoted by the addition of a base such as potassium *tert*-butoxide or potassium hydroxide.

The compounds of formula **I** wherein Y₁ and Y₄ represent CO can in general be obtained by reacting a compound of formula **III** with a cyclic anhydride of formula **VIII** 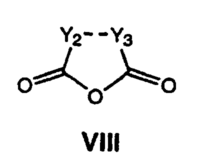 wherein Y₂ and Y₃ have the meaning described above.

The reaction is carried out in the presence of a base such as triethylamine, in a suitable solvent such as toluene or dimethoxyethane, and heating, preferably at reflux.

The compounds of formula **I** wherein one of Y₁ or Y₄ represents CH₂ and the other represents CO and the dotted line represents a double bond, can in general be obtained by reacting a compound of formula **III** with a compound of formula **IX** 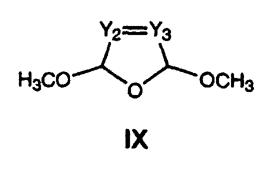 wherein Y₂ and Y₃ have the meaning described above.

The reaction is carried out in acidic medium, for example in the presence of hydrochloric acid, in a suitable solvent such as tetrahydrofuran/water mixtures, preferably at room temperature.

Some compounds of formula **I** can also be obtained by interconversion from another compound of formula **I,** for example by introduction of a substituent R₂ upon the corresponding compound **I** wherein ring A is unsubstituted, or by conversion of a substituent R₂ or R₆ into another group R₂ or R₆, respectively, in one or a plurality of steps, using standard reactions in heterocyclic chemistry, well known to those skilled in the art. Thus, for example, the compounds of formula **I** wherein R₂ and/or R₆ is halogen can be prepared from the corresponding compound **I** wherein ring A is unsubstituted or wherein R₆ = H, by treatment with a suitable halogenating agent such as a N-halosuccinimide or Br₂, as described in more detail in the examples.

All these interconversion reactions between substituents can be performed upon the final compounds as well as upon any synthetic intermediate thereof.

The salts of the compounds of formula **I** can be prepared by conventional procedures by treatment for example with an acid such as hydrochloric acid, sulfuric acid, nitric acid, oxalic acid or methanesulfonic acid or with a base such as sodium or potassium hydroxide, as described above.

The compounds of formulae **IV, V, VI, VI', VII, VIII** and **IX** are commercially available, widely described in the literature or can be prepared by methods analogous to those described using commercially available starting products.

The compounds of formula **II** are in general obtained by formation of ring A, using a large variety of methods depending on its structure.

The compounds of formula **II** wherein A is an imidazole (**IIa**) can be obtained as shown in scheme 1: 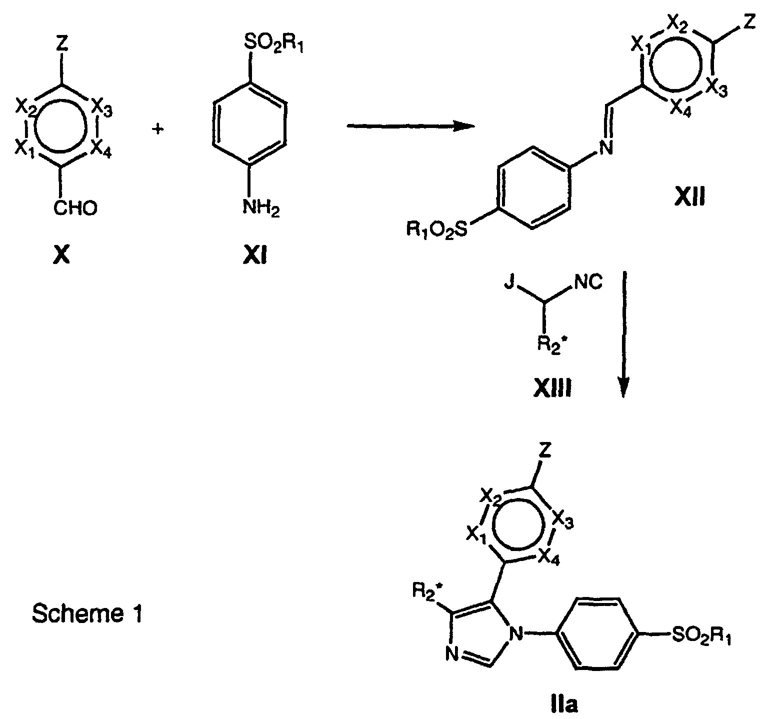 wherein R₂* represents hydrogen or R₂, and R₁, R₂, X₁, X₂, X₃, X₄ and Z have the meaning described above.

In the first step, an aldehyde of formula **X** is condensed with an amine of formula **XI**, heating at reflux in a suitable solvent such as benzene or toluene in a Dean Stark, to give a compound of formula **XII.**

In the second step, the imine obtained (**XII**) is reacted with an isocyanide of formula **XIII** wherein J represents a good leaving group such as a tosyl or 1*H*-benzotriazol-1-yl group. The reaction is carried out in the presence of a base such as K₂CO₃ in a suitable solvent such as methanol-dimethoxyethane mixtures, and heating, preferably at reflux. The isocyanides of formula **XIII** are commercially available, such as tosylmethylisocyanide and 1H-benzotriazol-1-ylmethylisocyanide, or can be prepared by alkylation of these with an alkyl iodide such as methyl iodide using the method described in the literature (A.M. van Leusen et al., *Tetrahedron Lett*. **1975**, 3487-88).

The aldehydes of formula **X** and the amines of formula **XI** are commercially available, widely described in the literature or can be prepared by methods analogous to those described using commercially available starting products. For example, an aldehyde of formula **X** can be prepared starting from the corresponding carboxylic acid in a sequence that comprises the conversion into an ester, for example an ethyl ester, under the standard conditions for the formation of esters, subsequent reduction of the ester to the alcohol with a suitable ester reducing agent such as lithium aluminum hydride, and finally oxidation of the alcohol to the aldehyde with a suitable oxidizing agent such as dimethylsulfoxide/oxalyl chloride.

This general procedure can be used to prepare other imidazoles different from **IIa** using appropriate starting compounds.

The compounds of formula **II** wherein A is a *5H*-furan-2-one (**IIb**) can be obtained as shown in scheme 2: 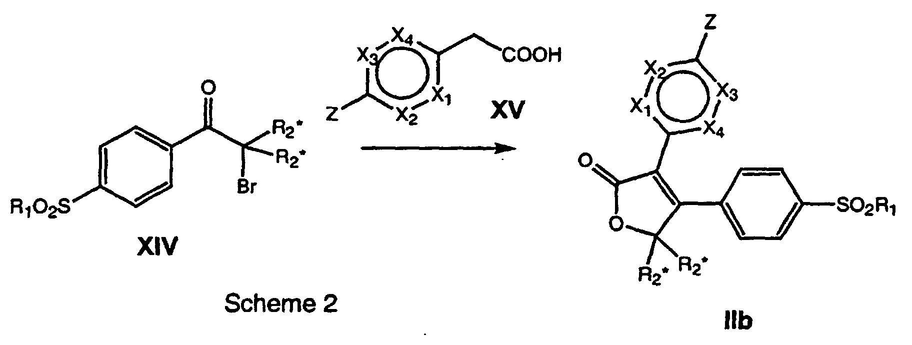 wherein R₁, R₂*, X₁, X₂, X₃, X₄ and Z have the meaning described above.

An arylbromomethylketone of formula **XIV** is firstly reacted with an arylacetic acid of formula **XV** in a suitable solvent such as acetonitrile, dimethylsulfoxide, dimethoxyethane or diethyl ether and in the presence of a base such as triethylamine or diisopropylethylamine, and next it is treated with 1,8-diazabicyclo[5.4.0]undec-7-ene (DBU) to give the furanone **IIb**.

The arylbromomethylketone of formula **XIV** can be easily obtained by halogenation of the corresponding acetophenone according to well-known methods in organic chemistry.

The compounds of formula **XV** are commercially available, widely described in the literature or they can be prepared by methods analogous to those described starting from commercially available starting products. For example, an arylacetic acid of formula **XV** can be prepared from the corresponding alcohol in a sequence that comprises firstly the conversion of the hydroxy group into a good leaving group, for example chloro, by treatment with thionyl chloride, followed by the introduction of a nitrile group by treatment with potassium cyanide in a suitable solvent such as methanol/water mixtures, and finally the hydrolysis of the nitrile group, for example in acidic medium, to give the carboxylic acid.

Alternatively, the compounds of formula **IIb** can also be prepared by reacting an α-hydroxyketone of formula **XVI** 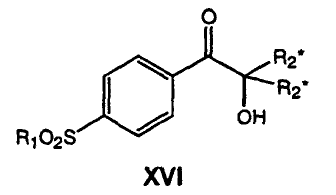 wherein R₁ and R₂* have the meaning described above, with an arylacetic acid of formula **XV,** in the presence of a coupling agent such as 1-cyclohexyl-3-(2-morpholinoethyl)carbodiimide and subsequent treatment with a base such as DBU.

The compounds of formula **XVI** can be prepared by methods described in the literature, for example from compounds of formula **XIV** by substitution of the bromo group with a hydroxy group according to methods well known to those skilled in the art.

The compounds of formula **II** wherein A is a pyrazole (**IIc**) can be obtained as shown in scheme 3: 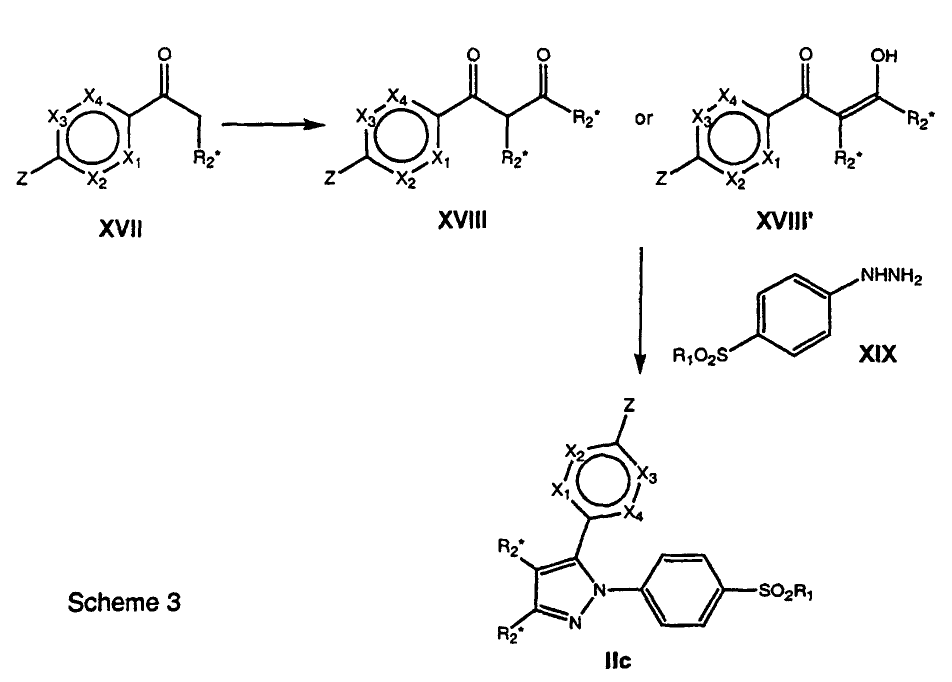 wherein R₁, R₂*, X₁, X₂, X₃, X₄ and Z have the meaning described above.

In a first step, an arylketone of formula **XVII** is treated with a base such as CH₃ONa, NaH or [(CH₃)₃Si]₂NLi, and an acylating agent, for example an ester R₂*COOR (wherein R = alkyl), in a suitable solvent such as *tert*-butyl methyl ether, diethyl ether, tetrahydrofuran, methanol or dichloromethane, to form the corresponding 1,3-diketone of formula **XVIII** or its equivalent **XVIII'.**

In a second step, the 1,3-diketone **XVIII** or its equivalent **XVIII'** is reacted with an hydrazine of formula **XIX,** as the free base or as a salt (for example the hydrochloride, hydrobromide, oxalate or sulfate), in an anhydrous protic solvent such as ethanol or acetic acid, and heating, preferably at reflux.

The arylketones of formula **XVII** and the hydrazines of formula **XIX** are commercially available, widely described in the literature or can be prepared by methods analogous to those described starting from commercially available starting products.

This general method for the synthesis of pyrazoles can be used to prepare other pyrazoles different from **IIc** using appropriate starting compounds.

The compounds of formula **II** wherein A is an oxazolone (**IId**) can be obtained as shown in scheme 4: 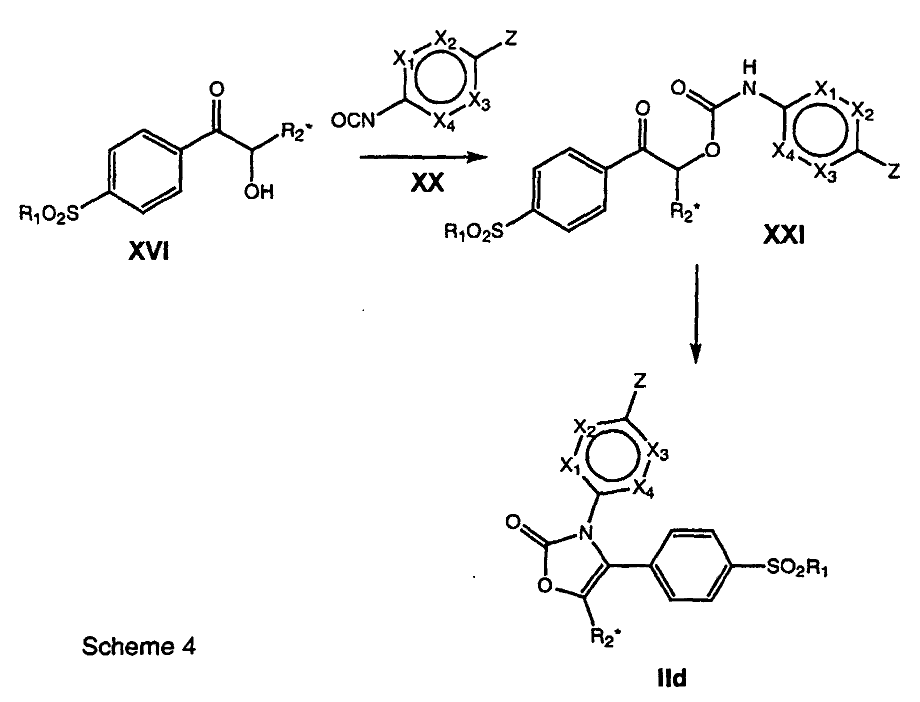 wherein R₁, R₂*, X₁, X₂, X₃, X₄ and Z have the meaning described above.

Firstly, an α-hydroxyketone of formula **XVI** is reacted with an arylisocyanate of formula **XX,** heating at a temperature preferably comprised between 80 and 200 °C and optionally in the presence of an organic solvent such as toluene or xylene, to give a compound of formula **XXI.** In a second step, the carbamate obtained (**XXI**) is treated with anhydrous acetic acid, heating, preferably at reflux, to give the oxazolone **IId**.

The arylisocyanates of formula **XX** are commercially available, widely described in the literature or can be prepared by methods analogous to those described using commercially available starting products.

This general method for the synthesis of oxazolones can be used to prepare other oxazolones different from **IId** using appropriate starting compounds.

The compounds of formula **II** wherein A is an oxazole (**IIe**) can be obtained as shown in scheme 5: 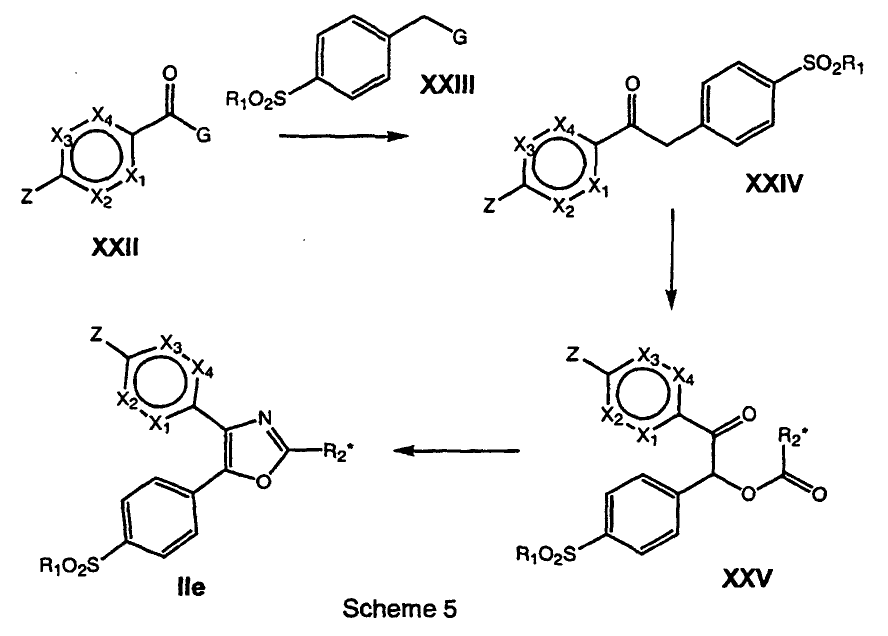 wherein R₁, R₂*, X₁, X₂, X₃, X₄ and Z have the meaning described above and G represents chloro or bromo.

In a first step, an acyl halide of formula **XXII** is reacted with a halogenated compound of formula **XXIII** to give a ketone of formula **XXIV.** The reaction is carried out in the presence of a metal such as zinc or magnesium, preferably zinc, in an inert solvent such as 1,2-dimethoxyethane, dioxane, diethyl ether, tetrahydrofuran, dichloromethane, benzene or toluene, preferably at a temperature comprised between room temperature and 50 °C. Optionally, the reaction can be carried out in the presence of a suitable catalyst such as Pd(PPh₃)₄.

In a second step the ketone obtained (**XXIV**) is reacted with a carboxylic acid of formula R2*COOH, in the presence of lead (IV) acetate or manganese (III) acetate, and optionally in the presence of a solvent such as benzene, toluene or xylene, at a temperature preferably comprised between 50 °C and reflux to give a ketone of formula **XXV.**

In the last step the oxazole is prepared by heating the compound of formula **XXV** in an alkylcarboxylic acid such as acetic acid, formic acid or propionic acid, in the presence of an ammonium salt such as ammonium acetate, ammonium formate or ammonium carbonate, preferably ammonium acetate.

The starting compounds of formula **XXII** and **XXIII** are commercially available, widely described in the literature or can be prepared by methods analogous to those described starting from commercially available starting products.

Alternatively, the ketone of formula **XXIV** can be prepared as shown in the following scheme: 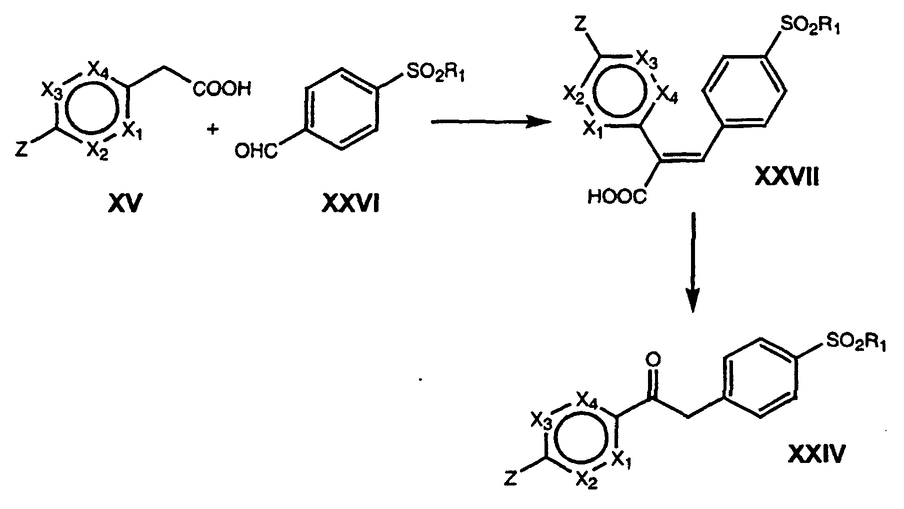 wherein R₁, X₁, X₂, X₃, X₄ and Z have the meaning described above.

In a first step, an acid of formula **XV** is reacted with an aldehyde of formula **XXVI,** in the presence of a base such as sodium methoxide and in a suitable solvent such as acetic anhydride. The reaction is preferably carried at the temperature of the boiling point of the solvent.

In a second step, the acid obtained (**XXVII**) is treated firstly with thionyl chloride, heating preferably at the temperature of the boiling point of the solvent, and then with sodium azide in an aqueous medium, at a temperature preferably comprised between 0 °C and room temperature. Finally it is heated in a mixture of acetic acid and water, preferably at reflux, to give the ketone of formula **XXIV.**

The aldehydes of formula **XXVI** are commercially available, widely described in the literature or can be prepared by methods analogous to those described starting from commercially available starting products, as described above for the aldehydes of formula **X.**

Alternatively, the α-carbonyloxyketone of formula **XXV** can be prepared from the corresponding α-hydroxyketone of formula **XXVIII** or α-haloketone of formula **XXIX** 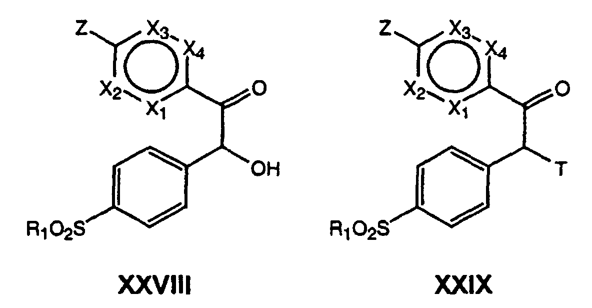 wherein R₁, X₁, X₂, X₃, X₄ and Z have the meaning described above, by reaction with an acyl halide of formula R₂*COT (wherein T represents halogen, as in the compounds of formula **XXIX**) or with a carboxylic acid of formula R₂*COOH respectively, in the presence of a base such as pyridine or triethylamine, in an inert solvent such as dichloromethane or chloroform, at a temperature comprised between -10 and 100 °C.

The α-hydroxyketone of formula **XXVIII** can be obtained by oxidation of the corresponding ketone of formula **XXIV,** obtained as described above, with an oxidizing agent such as iodobenzene diacetate.

The α-haloketone of formula **XXIX** can also be obtained from the corresponding ketone of formula **XXIV,** by halogenation with bromine, chlorine or N-bromosuccinimide, in a suitable solvent such as 1,2-dimethoxyethane, dioxane, diethyl ether, tetrahydrofuran, benzene or toluene.

This general method for the synthesis of oxazoles can be used to prepare other oxazoles using appropriate starting compounds.

The compounds of formula **II** wherein A is an isoxazole (**IIf**) can be obtained as shown in scheme 6: 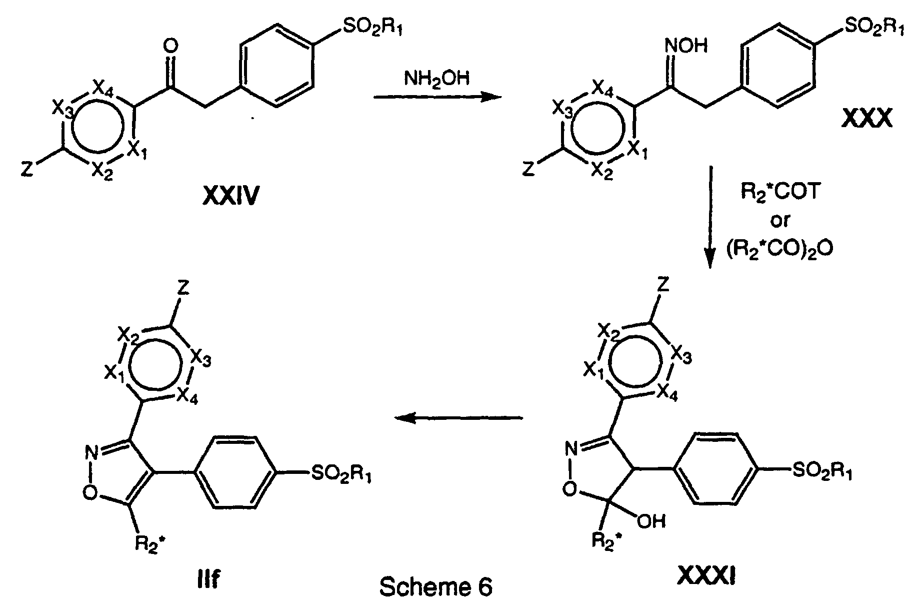 wherein R₁, R₂*, X₁, X₂, X₃, X₄ and Z have the meaning described above.

In a first step, the ketone of formula **XXIV,** obtained as described above, is reacted with hydroxylamine hydrochloride in the presence of a base such as sodium acetate or potassium hydroxide, in an inert solvent such as water, methanol, ethanol, isopropanol, tetrahydrofuran, 1,4-dioxane, diethyl ether, toluene, or mixtures thereof, at a temperature preferably comprised between 50 °C and reflux, to give an oxime of formula **XXX.**

Next, the oxime **XXX** is converted into a 4,5-dihydroisoxazole of formula **XXXI** by acylation with an acylating agent such as an acyl halide of formula R₂*COT or an anhydride of formula (R₂*CO)₂O, wherein R₂* and T have the meaning described above, in the presence of a base such as butyllithium, lithium diisopropylamide, lithium bis(trimethylsilyl)amide, sodium bis(trimethylsilyl)amide, or potassium bis(trimethylsilyl)amide. The reaction is carried out in an inert solvent such as tetrahydrofuran, diethyl ether, 1,2-dimethoxyethane, dioxane, benzene or dichloromethane, at a temperature preferably comprised between -78 °C and room temperature.

Finally, the isoxazole **IIf** can be obtained by dehydration of the 4,5-dihydroisoxazole obtained (**XXXI**), heating in the presence of an acid such as hydrochloric acid, hydrobromic acid, sulfuric acid, methanesulfonic acid, *p*-toluenesulfonic acid or polyphosphoric acid in a suitable solvent such as methanol, ethanol, isopropanol, tetrahydrofuran, diethyl ether, 1,4-dioxane, benzene, toluene, xylene, diglyme, dimethylformamide, dimethylsulfoxide or the like, at a temperature preferably comprised between 50 °C and 100 °C.

This general method for the synthesis of isoxazoles can be used to prepare other isoxazoles using appropriate starting compounds.

The compounds of formula **II** wherein A is a thiophene (**IIg**) can be obtained as shown in scheme 7: 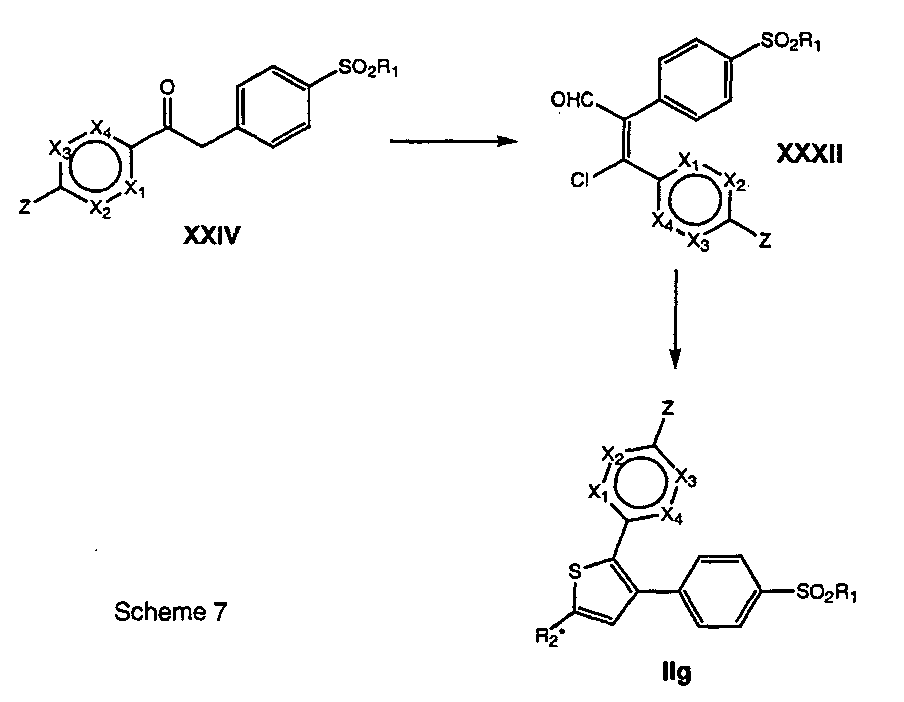 wherein R₁, R₂*, X₁, X₂, X₃, X₄ and Z have the meaning described above.

In a first step, the ketone of formula **XXIV,** obtained as described above, is reacted with the Vilsmeier's reagent (phosphorus oxychloride in dimethylformamide), preferably at room temperature, to give the β-chloroaldehyde of formula **XXXII.**

In a second step, the β-chloroaldehyde of formula **XXXII** is reacted with a mercaptoacetic acid of formula HS-CHR₂*-COOH in the presence of a base such as pyridine or triethylamine, and at a temperature comprised between room temperature and that of the boiling point of the solvent, to give the thiophene **IIg**.

This general method for the synthesis of thiophenes can be used to prepare other thiophenes using appropriate starting compounds.

The compounds of formula **II** wherein A is a cyclopentene (**IIh**) can be obtained as shown in scheme 8: 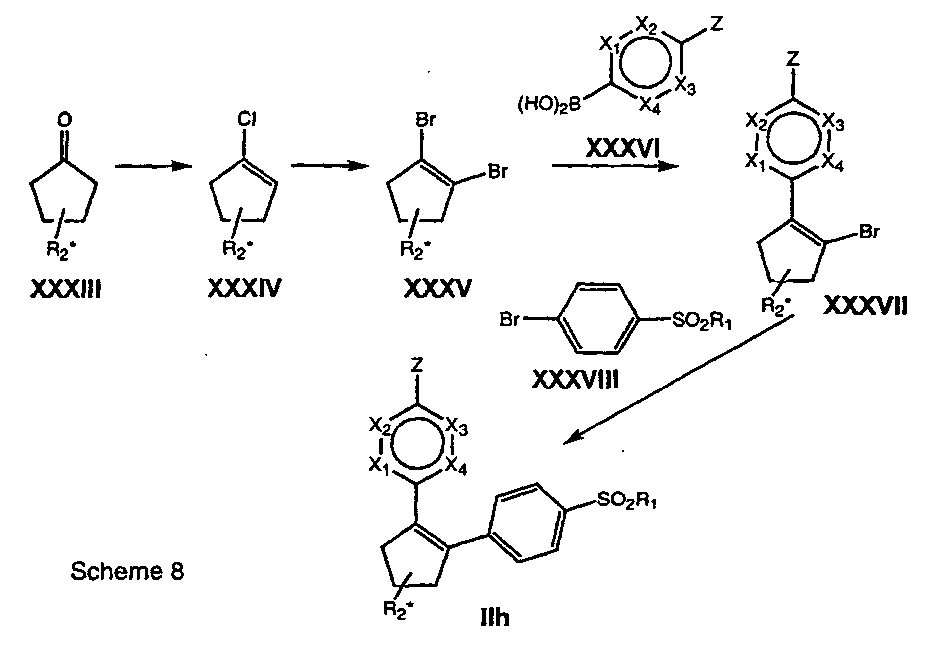 wherein R₁, R₂*, X₁, X₂, X₃, X₄ and Z have the meaning described above.

In a first step, a cyclopentanone of formula **XXXIII** is treated with phosphorus pentachloride in a suitable solvent such as toluene, preferably at room temperature, to give the 1-chlorocyclopentene of formula **XXXIV.**

Next, the 1-chlorocyclopentene **XXXIV** is treated with bromine, in the same solvent, at a temperature preferably comprised between -20 and 0 °C to give the 1,2-dibromocyclopentene of formula **XXXV.**

In a third step, the 1,2-dibromocyclopentene **XXXV** is reacted with an arylboronic acid of formula **XXXVI,** in the presence of a base such as sodium carbonate and a suitable catalyst such as Pd(PPh₃)₄, in a suitable solvent such as mixtures of toluene and ethanol, and heating, preferably at reflux, to give a compound of formula **XXXVII.**

The subsequent reaction of the compound **XXXVII** with a second arylboronic acid of formula (HO)₂B-C₆H₄-SO₂R₁ under the conditions described leads to the cyclopentene of formula **IIh**.

Alternatively, the cyclopentene of formula **IIh** can also be obtained from the compound of formula **XXXVII** by treatment with trimethyl borate in the presence of a base such as butyllithium, in a suitable solvent such as tetrahydrofuran, at a temperature preferably comprised between -78 °C and room temperature, and subsequent reaction with a halogenated compound of formula **XXXVIII,** in the presence of a base such as sodium carbonate and a suitable catalyst such as PdCl₂(PPh₃)₂, in a suitable solvent such as mixtures of tetrahydrofuran and water, and heating, preferably at reflux.

The cyclopentanones of formula **XXXIII**, the arylboronic acids of formula **XXXVI** and the compounds of formula **XXXVIII** are commercially available, widely described in the literature or can be prepared by methods analogous to those described starting from commercially available starting products. Thus, for example, the arylboronic acids of formula **XXXVI** can be obtained following the procedure described above for the last step of the synthesis of the compounds of formula **IIh**.

This general method for the synthesis of cyclopentenes can be used to prepare other cyclopentenes using appropriate starting compounds.

The compounds of formula **II** wherein A is a ring different from those specifically mentioned can be prepared according to the methods widely described in the literature for the preparation of said types of rings.

Alternatively, the compounds of formula **II** wherein R₁ represents C₁₋₈ alkyl or C₁₋₈ haloalkyl, can be obtained, independently from the structure of the central ring A, by oxidation of the corresponding tioether of formula **XXXIX:** 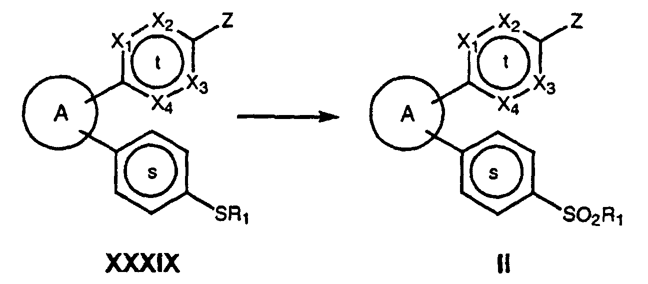 wherein A, X₁, X₂, X₃, X₄ and Z have the meaning described above and R₁ represents C₁₋₈ alkyl or C₁₋₈ haloalkyl.

The oxidation of the thioether to sulphone is carried out with a suitable oxidizing agent such as *m*-chloroperbenzoic acid, magnesium monoperoxyphthalate or Oxone® in a suitable solvent such as a halogenated hydrocarbon, for example dichloromethane.

Alternatively, the compounds of formula **II** wherein R₁ represents -NR₃R₄, wherein R₃ and R₄ have the meaning described above, can be obtained, independently of the structure of the central ring A, by introduction of the sulfonamide group (-SO₂NR₃R₄) on the corresponding benzene ring, as shown in scheme 9. 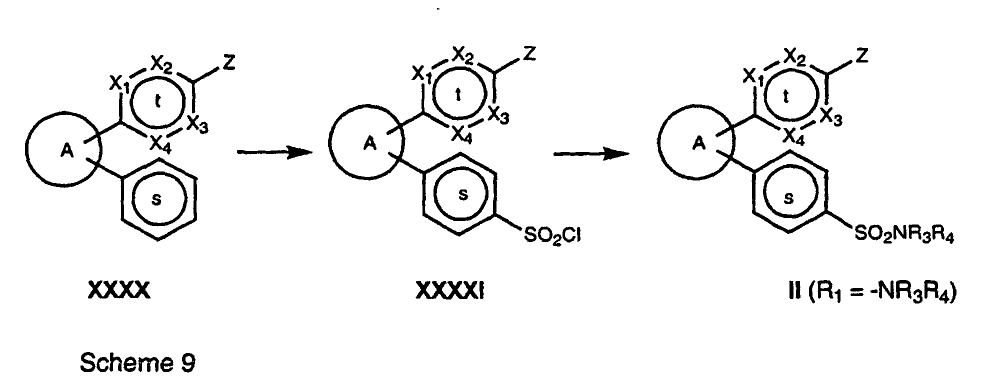 wherein A, X₁, X₂, X₃, X₄, R₃, R₄ and Z have the meaning described above.

In a first step, the chlorosulfonyl derivative of formula **XXXXI** is obtained by reacting a compound of formula **XXXX** with chlorosulfonic acid. Next, the chlorosulfonyl derivative **XXXXI** is reacted with an amine of formula NHR₃R₄, to give the corresponding sulfonamide.

Alternatively, the compounds of formula **II** wherein R₁ represents -NR₃R₄ can also be obtained from the corresponding methylthioether according to the sequence shown in scheme 10. 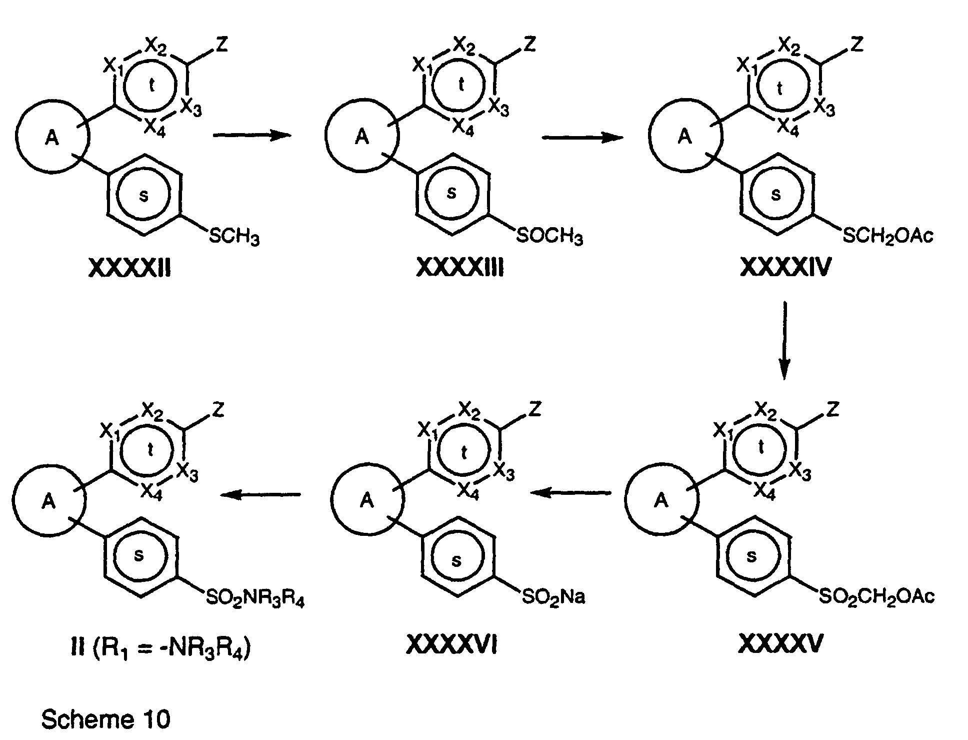 wherein X₁, X₂, X₃, X₄, R₃, R₄, A and Z have the meaning described above.

In a first step, a methylthioether of formula **XXXXII** is converted into the corresponding methylsulfoxide **XXXXIII** by oxidation with a suitable oxidizing agent such as *m*-chloroperbenzoic acid. Next, the methylsulfoxide **XXXXIII** is converted into the corresponding sodium sulfinate **XXXXVI** by a process that involves treatment with acetic anhydride to give the corresponding acetoxymethylthio derivative **XXXXIV,** which is oxidized with a suitable oxidizing agent such as magnesium monoperoxyphthalate to give the acetoxymethylsulfonyl derivative **XXXXV**, which is converted into the sodium sulfinate of formula **XXXXVI** by treatment with a base, for example sodium hydroxide. Finally, the unsubstituted sulfonamide (-SO₂NH₂) is obtained by reaction with hydroxylamine-O-sulfonic acid in a suitable solvent such as water or water/tetrahydrofuran mixtures, while the sulfonamide of formula -SO₂NR₃R₄ is obtained by chlorination with thionyl chloride followed by reaction with the corresponding amine HNR₃R₄.

The compounds of formulae **XXXIX, XXXX** and **XXXXII** can be obtained following procedures analogous to those described above for preparing the compounds of formula **II,** but starting from appropriate benzene derivatives.

The compounds of formula **III** are in general obtained using procedures analogous to those described above for preparing the compounds of formula **II,** but starting from compounds suitably substituted at the para position with a nitro group instead of a group Z, and subsequent reduction of the nitro group to an amino group, for example by hydrogenation in the presence of a suitable catalyst such as Pd/C or by treatment with a suitable reducing agent such as SnCl₂.

As mentioned above, the compounds of the present invention act by inhibiting the cyclooxygenase-2 enzyme (COX-2). Therefore, they are useful for the treatment or prevention of inflammation, pain and/or fever associated with a wide range of diseases or pathologies, which include among others: rheumatic fever; symptoms associated with influenza or other viral infections; common cold; low back and neck pain; dysmenorrhea; headache; toothache; myositis; neuralgia; synovitis; bursitis; arthritis, including rheumatoid arthritis and juvenile arthritis; degenerative joint diseases, including osteoarthritis; gout and ankylosing spondylitis; lupus erythematosus; tendinitis; sprains, strains and other similar injuries, such as those produced during sport performance; pain following surgical or dental procedures; and pain associated with cancer. They are also useful in the treatment of skin inflammatory diseases, including psoriasis, eczema, burns and dermatitis.

The compounds of the present invention can also be useful for the treatment of other pathologies mediated by COX-2. For example, the compounds of formula **I** can inhibit cell proliferation and consequently they can be useful for the treatment or prevention of familial adenomatous polyposis and cancer, especially those cancers that produce prostaglandins or that express cyclooxygenase. The compounds of the invention are useful for the treatment, for example, of liver, bladder, pancreas, ovary, prostate, cervix, lung, breast and skin cancer, and especially gastrointestinal cancers such as colon cancer.

The compounds of the present invention can also inhibit prostanoid-induced smooth muscle contraction and thus can be useful for the treatment of dysmenorrhea, preterm labour, asthma and bronchitis. Other uses of the compounds of formula **I** include the treatment or prevention of cerebral infarction, epilepsy, and neurodegenerative diseases, such as Alzheimer's disease and dementia.

Likewise, the compounds of the present invention can be used for treating inflammation in diseases such as vascular diseases, migraine, periarteritis nodosa, thyroiditis, aplastic anaemia, Hodgkin's disease, scleroderma, type **I** diabetes, myasthenia gravis, sarcoidosis, nephrotic syndrome, Behçet's syndrome, polymyositis, hypersensitivity, conjunctivitis, gingivitis and myocardial ischaemia.

Due to their selectivity for cyclooxygenase-2, the compounds of the present invention are useful as an alternative to non-steroidal anti-inflammatory drugs (NSAIDs), especially in those cases in which NSAIDs may be contra-indicated.

According to the activity of the products herein described, the present invention also relates to compositions which contain a compound of the present invention, together with an excipient or other auxiliary agents if necessary. The compounds of the present invention can be administered in the form of any pharmaceutical formulation, the nature of which will depend, as it is well known, upon the route of administration and the nature of the pathology to be treated.

According to the present invention, solid compositions for oral administration include tablets, powders for extemporaneous suspensions, granulates and capsules. In tablets, the active component is admixed with at least one inert diluent such as lactose, starch, mannitol or calcium phosphate; with a binding agent such as for example starch, gelatin, microcrystalline cellulose or polyvinylpyrrolidone; and with a lubricating agent, such as for example magnesium stearate, stearic acid or talc. Tablets can be coated by known techniques with the purpose of delaying their disintegration and absorption in the gastrointestinal tract, and thereby provide a sustained action over a longer period. Gastric or enteric coatings can be made with sugar, gelatin, hydroxypropylcellulose, acrylic resins, etc. Sustained-release tablets might also be obtained using an excipient which produces regressive osmosis, such as galacturonic acid polymers. Preparations for oral use can also be presented as hard capsules of absorbable material, such as for example gelatin, wherein the active compound is mixed with an inert solid diluent and lubricating agents, or pasty materials, such as ethoxylated saturated glycerides, which might also provide controlled release. Soft gelatin capsules are also possible, wherein the active compound is mixed with water or an oily medium, for example coconut oil, liquid paraffin, or olive oil.

Powders and granulates for the preparation of suspensions by the addition of water can be obtained by mixing the active compound with dispersing or wetting agents; suspending agents, such as sodium carboxymethylcellulose, methylcellulose, hydroxypropylmethylcellulose, sodium alginate, polyvinylpyrrolidine, gum tragacanth, xantham gum, gum acacia, and one or more preservatives, such as methyl or propyl *p*-hydroxybenzoate. Other excipients can also be added, for example sweetening, flavouring and colouring agents.

Liquid forms for oral administration include emulsions, solutions, suspensions, syrups and elixirs containing commonly-used inert diluents, such as distilled water, ethanol, sorbitol, glycerol or propylene glycols. Said compositions can also contain coadjuvants such as wetting, suspending, sweetening, flavouring, preserving agents and buffers.

Injectable preparations, according to the present invention, for parenteral administration comprise sterile aqueous or non-aqueous solutions, suspensions or emulsions, in a suitable non-toxic solvent or diluent. Examples of aqueous solvents or suspending media are distilled water for injection, Ringer's solution and isotonic sodium chloride solution. As non-aqueous solvents or suspending media propylene glycol, polyethylene glycol, vegetable oils such as olive oil, or alcohols such as ethanol can be used. These compositions can also contain coadjuvants, such as wetting, preserving, emulsifying and dispersing agents. They may be sterilized by any known method or prepared as sterile solid compositions to be dissolved in water or any other sterile injectable medium immediately before use. It is also possible to start from sterile materials and keep them under these conditions throughout all the manufacturing process.

The dosage and frequency of doses will depend upon the nature and severity of the disease to be treated, the age and body weight of the patient, as well as the route of administration. In general, the daily dose for an adult will be comprised between 1 and 1000 mg per day, which can be administered as a single or divided doses. However, in special cases, doses outside these margins might be necessary. A person skilled in the art will be able to easily determine the suitable dose for each situation.

Some examples of representative formulations for tablets, capsules and injectable preparations are given below. They can be prepared by conventional procedures and are useful for inhibiting cyclooxygenase-2.

| **Tablets** | | |
|---|---|---|
| Compound of formula **I** | 100 | mg |
| Dibasic calcium phosphate | 125 | mg |
| Sodium starch glycolate | 10 | mg |
| Talc | 12.5 | mg |
| Magnesium stearate | 2.5 | mg |
| | 250.0 | mg |

| **Hard gelatin capsules** | | |
|---|---|---|
| Compound of formula **I** | 100 | mg |
| Lactose | 197 | mg |
| Magnesium stearate | 3 | mg |
| | 300 | mg |

| **Injectable** | | |
|---|---|---|
| Compound of formula **I** | 100 | mg |
| Benzylic alcohol | 0.05 | mL |
| Propylene glycol | 1 | mL |
| Water to | 5 | mL |

The activity of the compounds of the present invention can be determined using the following test:

### Inhibition of cyclooxygenase-1 (COX-1) and cyclooxygenase-2 (COX-2) activity in human cell lines.

The inhibition of COX-1 and COX-2 is determined by assessing the PGE₂ production after stimulation with arachidonic acid in cell lines expressing human COX-1 (U-937 from human histiocitic lymphoma) and human COX-2 (143.98.2 from human osteosarcoma), respectively.

The osteosarcoma-derived cells were cultured in 1 mL of DMEM culture medium supplemented with 10% fetal calf serum, in 24-well multidishes until confluence. U-937 cells were cultured in RPMI medium supplemented with 10% fetal calf serum in flasks.

To evaluate COX-2 activity, the medium was removed and replaced with Hepes-buffered saline solution (HBSS) without Ca²⁺/Mg²⁺ (2 x 10⁵ cells/well). To evaluate COX-1 activity, the medium was removed and U-937 cells were resuspended to a final density of 3 x 10⁶ cells/mL in HBSS without Ca²⁺/Mg²⁺ (1 mL/well, in 24-well multidishes). 1 µL of a solution of the test compound dissolved in DMSO or vehicle was added, and the samples were incubated for 15 min at 37 °C (5% CO₂ and 95% humidity). Arachidonic acid was added (final concentration: 10 µM) and the samples were incubated for 10 min more. Next the reactions were quenched by adding indomethacin (8 mM, 30 µL). The amount of PGE₂ in the supernatant was determined by specific enzymatic immunoassay (Kit Prostaglandin E2, Biotrak EIA system RPN222, Amersham Pharmacia Biotech). All the assays were performed in triplicate.

The results obtained with representative compounds of the present invention are shown in the following table, where the % of inhibition of COX-1 and COX-2 activity at a concentration of 0.1 µM of test compound are reported.

| Example | **% Inhibition (0.1 µM)** | |
|---|---|---|
| | COX-1 | COX-2 |
| 1 | 0 | 61 |
| 3 | 11 | 75 |
| 4 | 0 | 72 |
| 5 | 0 | 70 |
| 6 | 0 | 89 |
| 7 | 0 | 72 |
| 8 | 0 | 76 |
| 9 | 0 | 72 |
| 10 | 0 | 61 |
| 12 | 0 | 79 |
| 14 | 0 | 49 |
| 15 | 17 | 81 |
| 16 | 0 | 93 |
| 17 | 0 | 81 |

The results of the table above show that the compounds of formula **I** are potent and selective COX-2 inhibitors.

The following examples illustrate, but do not limit, the scope of the present invention. The following abbreviations have been used in the examples:
EtOAc: ethyl acetate
Ac₂O: acetic anhydride
NaOAc: sodium acetate
BuLi: butyllithium
DBU: 1,8-diazabicyclo[5.4.0]undec-7-ene
DME: dimethoxyethane
DMF: dimethylformamide
DMSO: dimethylsulfoxide
EtOH: ethanol
Et₂O: diethyl ether
MCPBA: *m*-chloroperbenzoic acid
MeOH: methanol
NaOMe: sodium methoxide
Et₃N: triethylamine
THF: tetrahydrofuran
TMS: tetramethylsilane

### Reference example 1

### 5-(4-Fluorophenyl)-1-(4-methylsulfonylphenyl)imidazole

### a) 4-Methylsulfonylaniline

67 mg of Na₂WO₄, 8 drops of acetic acid and 19 mL of H₂O were placed in a flask and heated to 65 °C. Then, 19 mL (153 mmol) of 4-methylthioaniline was added followed by 34.5 mL (337 mmol) of H₂O₂ dropwise. The mixture was stirred at 65 °C for 1.5 h and, after cooling, 800 mL of 1 N HCl and 500 mL of CHCl₃ were added. The layers were separated and the aqueous phase was washed with more CHCl₃. The aqueous phase was basified with 25% NaOH and extracted with CHCl₃. The organic phase was washed with brine and dried over MgSO₄. The solvent was removed, yielding 19.80 g of the product as a white solid (75% yield).
¹H-NMR (300 MHz, CDCl₃ δ TMS): 2.97 (s, 3 H), 4.04 (s, 2 H), 6.66 (d, J = 9 Hz, 2 H), 7.56 (d, J = 9 Hz, 2 H).

### b) N-(4-Fluorobenzyliden)-4-methylsulfonylaniline

A mixture of 19.60 g (115 mmol) of 4-methylsulfonylaniline (obtained in the preceding section), 12.19 mL (115 mmol) of 4-fluorobenzaldehyde and 590 mL of toluene was refluxed in a Dean-Stark for 2 days. The solvent was removed and the crude product obtained was directly used in the next reaction.

A sample was recrystallized from Et₂O to give the analytically-pure compound.
¹H-NMR (300 MHz, CDCl₃ δ TMS): 3.08 (s, 3 H), 7.20 (m, 2 H), 7.30 (m, 2 H), 7.98 (m, 4 H), 8.38 (s, 1 H).

### c) Title compound

A mixture of 31.8 g (115 mmol) of N-(4-fluorobenzyliden)-4-methylsulfonylaniline (obtained in the preceding section), 33.4 g (172 mmol) of tosylmethylisocyanide, 31.7 g (229 mmol) of K₂CO₃, 795 mL of MeOH and 340 mL of DME was refluxed for 2 h. The solvent was removed and the residue was redissolved in a CH₂Cl₂/brine mixture and the layers were separated. The aqueous phase was extracted with CH₂Cl₂ and the combined organic phases were dried over MgSO₄ and concentrated. A crude product was obtained, which was washed with Et₂O several times to give 29.0 g of a creamy solid. Finally it was recrystallized from EtOAc/hexane (120/25 mL), yielding 27.2 g of the product as a creamy solid (75% yield).
¹H-NMR (300 MHz, CDCl₃ δ TMS): 3.10 (s, 3 H), 7.05 (m, 2 H), 7.13 (m, 2 H), 7.26 (s, 1 H), 7.36 (d, J = 9 Hz, 2 H), 7.75 (s, 1 H), 7.99 (d, J = 9 Hz, 2 H).

### Reference example 2

### 4-Chloro-5-(4-fluorophenyl)-1-(4-methylsulfonylphenyl)imidazole

A mixture of 27.2 g (86 mmol) of 5-(4-fluorophenyl)-1-(4-methylsulfonylphenyl)imidazole (obtained in reference example 1), 12.05 g (90 mmol) of N-chlorosuccinimide and 81 mL of CHCl₃ was refluxed for 18 h. The solvent was removed and the residue was redissolved in CH₂Cl₂ and washed with 1 N HCI and next with 1 N NaOH and brine. The organic phase was dried over MgSO₄ and concentrated. The crude product obtained was washed with Et₂O several times to afford 26.2 g of a creamy solid, which was purified by chromatography on silica gel, using EtOAc/hexane mixtures of increasing polarity as eluent. 24.0 g of the title compound of the example was obtained as a white solid (80% yield).
¹H-NMR (300 MHz, CDCl₃ δ TMS): 3.13 (s, 3 H), 7.12 (m, 2 H), 7.20 (m, 2 H), 7.32 (d, J = 9 Hz, 2 H), 7.71 (s, 1 H), 8.02 (d, J = 9 Hz, 2 H).

### Reference example 3

### 5-(4-Aminophenyl)-4-chloro-1-(4-methylsulfonylphenyl)imidazole

### a) 1-(4-Methylsulfonylphonyl)-5-(4-nitrophonyl)imidazole

Following a similar procedure to that described in reference example 1, but using 4-nitrobenzaldehyde instead of 4-fluorobenzaldehyde, the desired compound was obtained (84% yield).
¹H-NMR (300 MHz, CDCl₃ δ TMS): 3.09 (s, 3 H), 7.31 (d, J = 8.5 Hz, 2 H), 7.44 (d, J = 8.5 Hz, 2 H), 7.52 (s, 1 H), 7.87 (s, 1 H), 8.10 (d, J = 8.5 Hz, 2 H), 8.23 (d, J = 8.5 Hz, 2 H).

### b) 4-Chloro-1-(4-methylsulfonylphenyl)-5-(4-nitrophenyl)imidazole

Following a similar procedure to that described in reference example 2, but starting from the product obtained in the preceding section, the desired compound was obtained (56% yield).
¹H-NMR (300 MHz, CDCl₃ δ TMS): 3.09 (s, 3 H), 7.34 (d, J = 8.5 Hz, 2 H), 7.39 (d, J = 8.5 Hz, 2 H), 7.72 (s, 1 H), 8.02 (d, J = 8.5 Hz, 2 H), 8.20 (d, J = 8.5 Hz, 2 H).

### c) Title compound

A mixture of 1.14 g (3 mmol) of 4-chloro-1-(4-methylsulfonylphenyl)-5-(4-nitrophenyl)imidazole (obtained in the preceding section), 2.88 g (15 mmol) of SnCl₂ and 21 mL of EtOH was refluxed for 1.5 h. The solvent was removed and the residue was basified with 25% NaOH and extracted with CHCl₃. The organic phase was dried over MgSO₄ and concentrated. The residue was purified by chromatography on silica gel using hexane/EtOAc mixtures of increasing polarity as eluent. 0.855 g of the product was obtained as a yellow solid (81% yield).
¹H-NMR (300 MHz, CDCl₃ + CD₃OD δ TMS): 3.08 (s, 3 H), 4.0 (s, 2 H + H₂O), 6.60 (d, J = 8.5 Hz, 2 H), 6.90 (d, J = 8.5 Hz, 2 H), 7.35 (d, J = 8.5 Hz, 2 H), 7.66 (s, 1 H), 7.93 (d, J = 8.5 Hz, 2 H).

### Reference example 4

### 5-(6-Chloro-3-pyridyl)-1-(4-methylsulfonylphenyl)imidazole

### a) 6-Chloronicotinic acid ethyl ester

A mixture of 5 g (31.7 mmol) of 6-chloronicotinic acid and 16 mL of SOCl₂ was heated at reflux under argon for 1 h. The solvent was removed and the residue was stirred with a mixture of 8.9 mL of Et₃N and 130 mL of ethanol for 1.5 h at room temperature. The solvent was removed and the residue was partitioned between CH₂Cl₂ and H₂O. The phases were separated and the aqueous one was extracted with CH₂Cl₂. The combined organic extracts were dried over MgSO₄ and concentrated, affording 5.78 g of a crude product that was used in the following step.
¹H-NMR (300 MHz, CDCl₃ δ TMS): 1.40 (t, J = 7.2 Hz, 3 H), 4.41 (q, J = 7.2 Hz, 2 H), 7.41 (d, J = 8.4 Hz, 1 H), 8.24 (d, J = 8.4 Hz, 1 H), 8.98 (s, 1 H).

### b) 6-Chloro-3-pyridylmethanol

To a mixture of 2.36 g (62.28 mmol) of LiAlH₄ and 190 mL of Et₂O under argon, 5.8 g (31.14 mmol) of 6-chloronicotinic acid ethyl ester (obtained in the preceding section) dissolved in 380 mL of Et₂O was added and the resulting mixture was stirred at room temperature overnight. A mixture of 3.85 mL of H₂O and 8.12 mL of THF, followed by 3.85 mL of 15% NaOH, and then 10.54 mL of H₂O were added dropwise and finally Na₂SO₄ was added. The resulting mixture was filtered, washed with Et₂O and EtOAc and the solvent was evaporated. A crude product was obtained, which was purified by chromatography on silica gel using EtOAc/hexane mixtures of increasing polarity as eluent to give 1.71 g of the desired product (38% yield).
¹H-NMR (300 MHz, CDCl₃ δ TMS): 2.60 (t, J = 5.7 Hz, 1 H), 4.74 (d, J = 5.7 Hz, 2 H), 7.33 (d, J = 8.1 Hz, 1 H), 7.71 (d, J = 8.1 Hz, 1 H), 8.35 (s, 1 H).

### c) 6-Chloro-3-pyridincarbaldehyde

To a mixture of 1.14 mL (13.04 mmol) of oxalyl chloride and 16.6 mL of CH₂Cl₂, a mixture of 2.0 mL of DMSO and 3.8 mL of CH₂Cl₂ was added at -78 °C and under argon, and the resulting mixture was stirred for 5 min. Then, 1.71 g (11.91 mmol) of 6-chloro-3-pyridylmethanol (obtained in the preceding section) dissolved in a mixture of 1.5 mL of DMSO and 1.5 mL of CH₂Cl₂ was added dropwise and the mixture was stirred for 30 min at -78 °C. 14.2 mL (105.04 mmol) of Et₃N was added and the mixture was stirred for 10 min at the same temperature and then it was allowed to warm up to room temperature. The resulting mixture was poured into a mixture of ice and H₂O, extracted with CH₂Cl₂ and the combined organic phases were dried over MgSO₄, filtered and concentrated, affording 1.4 g of the desired product (83% yield).
¹H-NMR (300 MHz, CDCl₃ δ TMS): 7.49 (d, J = 8.1 Hz, 1 H), 8.11 (d, J = 8.1 Hz, 1 H), 8.83 (s, 1 H), 10.07 (s 1 H).

### d) N-(6-Chloro-3-pyridylmethyliden)-4-methylsulfonylaniline

Following a similar procedure to that described in section b of reference example 1, but starting from 6-chloro-3-pyridincarbaldehyde (obtained in the preceding section) instead of 4-fluorobenzaldehyde and using benzene as solvent instead of toluene, a crude product was obtained which was directly used in the following reaction.
¹H-NMR (300 MHz, CDCl₃ + CD₃OD δ TMS): 2.98 (s, 3 H), 5.64 (s, 1 H), 6.83 (d, J = 8.1 Hz, 2 H), 7.38 (d, J = 8.1 Hz, 1 H), 7.64 (d, J = 8.1 Hz, 2 H), 7.84 (d, J = 8.1 Hz, 1 H), 8.45 (s, 1 H).

### e) Title compound

Following a similar procedure to that described in section c of reference example 1 but starting from N-(6-chloro-3-pyridylmethyliden)-4-methylsulfonylaniline (obtained in the preceding section) instead of N-(4-fluorobenzyliden)-4-methylsulfonylaniline, a crude product was obtained which was purified by chromatography on silica gel using EtOAc/hexane mixtures of increasing polarity as eluent. The title compound was obtained in 59% yield.
¹H-NMR (300 MHz, CDCl₃ δ TMS): 3.12 (s, 3 H), 7.27 (s, 1 H), 7.30 (d, 1 H), 7.40 (m, 3 H), 7.82 (s, 1 H), 8.05 (d, J = 8.4 Hz, 2 H), 8.21 (s, 1 H).

### Reference example 5

### 4-[4-Chloro-5-(4-fluorophenyl)imidazol-1-yl]benzenesulfonamide

### a) 4-Methylsulfinylaniline

8.9 mL (71.8 mmol) of 4-methylthioaniline and 330 mL of CH₂Cl₂ were placed in a flask, the mixture was cooled to 0 °C, 22.5 g (71.8 mmol) of MCPBA was then added and the mixture was stirred at room temperature for 2 h. CHCl₃ and saturated NaHCO₃ solution were added, the mixture was saturated with solid NaCI and the layers were separated. The aqueous phase was extracted with more CHCl₃ and the combined organic phases were dried over MgSO₄ and concentrated. The crude product obtained was purified by chromatography on silica gel, using EtOAc/hexane mixtures of increasing polarity as eluent to give 9 g of the desired product (80% yield).
¹H-NMR (300 MHz, CDCl₃ δ TMS): 2.68 (s, 3 H), 4.02 (s, broad signal, 2 H), 6.75 (d, J = 8.7 Hz, 2 H), 7.45 (d, J = 8.7 Hz, 2 H).

### b) N-(4-Fluorobenzyliden)-4-methylsulfinylaniline

Following a similar procedure to that described in section b of reference example 1, but starting from 4-methylsulfinylaniline (obtained in the preceding section) instead of 4-methylsulfonylaniline, and using benzene as solvent instead of toluene, a crude product was obtained which was directly used in the following reaction.
¹H-NMR (300 MHz, CDCl₃ δ TMS): 2.75 (s, 3 H), 7.18 (m, 2 H), 7.32 (m, 2 H), 7.68 (d, J = 8.5 Hz, 2 H), 7.91 (m, 2 H), 8.41 (s, 1 H).

### c) 5-(4-Fluorophenyl)-1-(4-methylsulfinylphenyl)imidazole

Following a similar procedure to that described in section c of reference example 1, but starting from N-(4-fluorobenzyliden)-4-methylsulfinylaniline (obtained in the preceding section) instead of N-(4-fluorobenzyliden)-4-methylsulfonylaniline, a crude product was obtained which was washed with Et₂O several times to give the product as a creamy solid (60% yield).
¹H-NMR (300 MHz, CDCl₃ δ TMS): 2.77 (s, 3 H), 6.99 (m, 2 H), 7.10 (m, 2 H), 7.25 (s, 1 H), 7.33 (d, J = 8.5 Hz, 2 H), 7.69 (d, J = 8.5 Hz, 2 H), 7.73 (s, 1 H).

### d) 1-[4-(Acetoxymethylsulfanyl)phenyl]-5-(4-fluorophenyl)imidazole

1.60 g (5.3 mmol) of 5-(4-fluorophenyl)-1-(4-methylsulfinylphenyl)imidazole (obtained in the preceding section), 16 mL of Ac₂O and 1.6 g (20 mmol) of NaOAc were placed in a flask under a nitrogen atmosphere and the mixture was heated at reflux for 8 h. The solvent was removed and the crude product was purified by chromatography on silica gel using EtOAc/hexane mixtures of increasing polarity as eluent, affording 1.6 g of the product as a foamy solid (84% yield).
¹H-NMR (300 MHz, CDCl₃ δ TMS): 2.11 (s, 3 H), 5.44 (s, 2 H), 6.99 (m, 2 H), 7.10 (m, 2 H), 7.14 (s, 1 H), 7.25 (d, J = 8.5 Hz, 2 H), 7.47 (d, J = 8.5 Hz, 2 H), 7.72 (s, 1H).

### e) 1-[4-(Acetoxymethylsulfanyl)phenyl]-4-chloro-5-(4-fluorophenyl)imidazole

Following a similar procedure to that described in reference example 2 but starting from 1-[4-(acetoxymethylsulfanyl)phenyl]-5-(4-fluorophenyl)imidazole (obtained in the preceding section), the desired compound was obtained in 51% yield.
¹H-NMR (300 MHz, CDCl₃ δ TMS): 2.11 (s, 3 H), 5.43 (s, 2 H), 7.05 (m, 4 H), 7.19 (m, 2 H), 7.44 (d, J = 8.6 Hz, 2 H), 7.59 (s, 1 H).

### f) Sodium 4-[4-chloro-5-(4-fluorophonyl)imidazol-1-yl]benzenesulflnate

The product obtained in the preceding section, 8 mL of CH₂Cl₂ and 4 mL of MeOH were placed in a flask and the mixture was cooled to 0 °C. 1.5 g (2.6 mmol) of magnesium monoperoxyphthalate hexahydrate was added and the mixture was stirred overnight at room temperature. 12 mL of 5% NaHCO₃ was added and the mixture was extracted with CH₂Cl₂. The solvent was removed and the residue was dissolved in a mixture of 8 mL of THF and 4 mL of MeOH and was cooled to 0 °C. 2.56 mL of 1 N NaOH was added and the resulting mixture was stirred for 1 h at room temperature. It was concentrated, H₂O was removed by azeotropic distillation with EtOH/toluene mixtures, and the residue was dried *in vacuo*. 0.90 g of a crude product was obtained, which was directly used in the following step.
¹H-NMR (300 MHz, CDCl₃ + CD₃OD δ TMS): 6.99 (m, 2 H), 7.18 (m, 4 H), 7.63 (s, 1 H), 7.68 (d, J = 8.2 Hz, 2 H).

### g) Title compound

The crude product obtained in the preceding section, 13 mL of H₂O, 0.21 g (2.7 mmol) of NaOAc and 0.30 g (2.7 mmol) of hydroxylamine-O-sulfonic acid were placed in a flask and the mixture was stirred overnight at room temperature. The suspension obtained was filtered and the solid was washed with EtOAc and H₂O. The layers were separated and the aqueous phase was extracted with EtOAc. The combined organic phases were dried over MgSO₄ and concentrated. The residue was purified by chromatography on silica gel using hexane-EtOAc mixtures of increasing polarity as eluent, affording 0.420 g of the product as a yellow solid (48 % yield).
¹H-NMR (300 MHz, CDCl₃ δ TMS): 4.84 (s, 2 H), 7.05 (m, 2 H), 7.18 (m, 2 H), 7.26 (d, J = 8.7 Hz, 2 H), 7.65 (s, 1 H), 7.96 (d, J = 8.7 Hz, 2 H).

### Reference example 6

### 3-(4-Aminophenyl)-4-(4-methylsulfonylphenyl)-5H-furan-2-one

### a) 2-Bromo-1-(4-methylsulfonylphenyl)ethanone

To a solution of 8.2 g (41.3 mmol) of 1-(4-methylsulfonylphenyl)ethanone in 100 mL of CHCl₃, cooled to -5 °C and under argon, 0.8 mg of AlCl₃ and 1.78 mL (34.5 mmol) of Br₂ in 12.2 mL of CHCl₃ were added. The mixture was allowed to warm up to room temperature, H₂O was added and the layers were separated. The aqueous phase was extracted with EtOAc, and the combined organic phases were dried over MgSO₄ and concentrated. 11 g of a crude product was obtained, which was directly used in the following reaction.
¹H-NMR (300 MHz, CDCl₃ δ TMS): 3.10 (s, 3 H), 4.46 (s, 2 H), 8.08 (d, J = 8.7 Hz, 2 H), 8.17 (d, J=8.7 Hz, 2 H).

### b) 4-(4-Methylsulfonylphenyl)-3-(4-nitrophenyl)-5H-furan-2-one

To a mixture of 10.74 g (38.7 mmol) of the compound obtained in the preceding section, 121 mL of acetonitrile and 6.23 g (34.4 mmol) of 4-nitrophenylacetic acid, 12.08 mL (86.7 mmol) of Et₃N was added dropwise and under argon, and the resulting mixture was stirred for 1 h at room temperature. It was cooled to 0 °C, 10.06 mL (67.3 mmol) of DBU was added and the mixture was stirred for 2 h at this temperature. Then, 121 mL of 1 N HCI was added, the mixture was extracted with EtOAc, and the organic phase was dried over MgSO₄ and concentrated. The crude product obtained was purified by chromatography on silica gel using EtOAc/hexane mixtures of increasing polarity as eluent, yielding 8 g of the desired compound (60% yield).
¹H-NMR (300 MHz, CDCl₃ δ TMS): 3.10 (s, 3 H), 5.25 (s, 2 H), 7.50 (d, J = 8.7 Hz, 2 H), 7.62 (d, J = 8.7 Hz, 2 H), 7.99 (d, J = 8.7 Hz, 2 H), 8.28 (d, J = 8.7 Hz, 2 H).

### c) Title compound

A mixture of 4.0 g (11.14 mmol) of 4-(4-methylsulfonylphenyl)-3-(4-nitrophenyl)-*5H*-furan-2-one (obtained in the preceding section), 75 mL of EtOH, 75 mL of THF and 0.63 g of 10% Pd/C was stirred for 2 h under a hydrogen atmosphere. The resulting mixture was filtered through celite and concentrated. The title compound of the example was obtained as a yellow solid (95% yield).
¹H-NMR (300 MHz, CDCl₃ + CD₃OD δ TMS): 3.11 (s, 3 H), 3.69 (s, 2 H), 5.18 (s, 2 H), 6.69 (d, J = 8.7 Hz, 2 H), 7.21 (d, J = 8.7 Hz, 2 H), 7.60 (d, J = 8.7 Hz, 2 H), 7.93 (d, J = 8.7 Hz, 2 H).

### Reference example 7

### 4-[5-(4-Aminophenyl)-3-trifluoromethyl-1H-pyrazol-1-yl]benzenesulfonamide

### a) 4,4,4-Trifluoro-1-(4-nitrophenyl)-1,3-butanedione

To a solution of 2.3 mL (20 mmol) of ethyl trifluoroacetate in 9.4 mL of *tert*-butyl methyl ether, a solution of NaOMe in MeOH previously prepared (0.56 g of Na in 4.7 mL of MeOH) and 3 g (18.16 mmol) of 4-nitroacetophenone were added under argon. The mixture was stirred at room temperature overnight, and then 8.4 mL of 3 N HCI was added. The suspension obtained was extracted with EtOAc and the organic phase was dried over MgSO₄. The solvent was removed and the crude product obtained was purified by chromatography on silica gel using EtOAc/hexane mixtures of increasing polarity as eluent, affording 4.2 g of the desired compound (88% yield).
¹H-NMR (300 MHz, CDCl₃ δ TMS): 6.62 (s, 1 H), 7.12 (d, J = 9.0 Hz, 2 H), 7.26 (s, 1 H), 8.37 (d, J = 9.0 Hz, 2 H).

### b) 4-[3-Trifluoromethyl-5-(4-nitrophenyl)-1H-pyrazol-1-yl]benzenesulfonamide

A mixture of 4.2 g (16 mmol) of 4,4,4-trifluoro-1-(4-nitrophenyl)-1,3-butanedione (obtained in the preceding section), 200 mL of EtOH and 3.9 g (17.58 mmol) of 4-sulfamoylphenylhydrazine hydrochloride was heated at reflux overnight under argon. The resulting mixture was concentrated, EtOAc was added and the mixture was washed with H₂O and brine. It was then dried over MgSO₄, concentrated and the crude product obtained was washed with CHCl₃, affording 6.1 g of the desired compound (100% yield).
¹H-NMR (300 MHz, CDCl₃ + CD₃OD δ TMS): 6.95 (s, 1 H), 7.46 (d, J = 8.7 Hz, 4 H), 7.97 (d, J = 8.7 Hz, 2 H), 8.26 (d, J = 8.7 Hz, 2 H).

### c) Title compound

Following a similar procedure to that described in section c of reference example 6, but starting from 4-[3-trifluoromethyl-5-(4-nitrophenyl)-1H-pyrazol-1-yl]benzenesulfonamide (obtained in the preceding section), and allowing the reaction to proceed for 4 h instead of 2 h, the title compound was obtained in quantitative yield.
¹H-NMR (300 MHz, CDCl₃ + CD₃OD δ TMS): 6.59 (d, J = 8.7 Hz, 2 H), 6.64 (s, 1 H), 6.93 (d, J = 8.7 Hz, 2 H), 7.43 (d, J = 8.7 Hz, 2 H), 7.87 (d, J = 8.7 Hz, 2 H).

### Reference example 8

### 6-(1-Pyrrolidinyl)-3-pyridylacetic acid

### a) 6-Chloro-3-pyridylacetonitrile

A mixture of 0.5 g (3.48 mmol) of 6-chloro-3-pyridylmethanol (obtained in section b of reference example 4) and 3.48 mL (48 mmol) of SOCl₂ was heated at reflux for 3 h. It was then cooled, benzene was added and the resulting mixture was concentrated. The crude product obtained was treated with 0.64 g (9.74 mmol) of KCN, 5.2 mL of MeOH and 2 mL of H₂O and the resulting mixture was heated at reflux for 2 h. Then, 5 mL of H₂O was added, the mixture was saturated with K₂CO₃ and was extracted with Et₂O. The organic phase was dried over MgSO₄ and concentrated. The crude product obtained was purified by chromatography on silica gel using EtOAc/hexane mixtures of increasing polarity as eluent, affording 0.293 g of the desired compound (55% yield).
¹H-NMR (300 MHz, CDCl₃ δ TMS): 3.77 (s, 2 H), 7.38 (d, J = 8.1 Hz, 1 H), 7.68 (d, J = 8.1 Hz, 1 H), 8.36 (s, 1 H).

### b) 6-(1-Pyrrolidinyl)-3-pyridylacetonitrile

A mixture of 0.293 g (1.92 mmol) of 6-chloro-3-pyridylacetonitrile (obtained in the preceding section) and 9.3 mL of pyrrolidine was refluxed for 2 h under argon. The mixture was allowed to cool and was concentrated, and the residue was partitioned between EtOAc and H₂O. The layers were separated and the organic phase was dried over MgSO₄ and concentrated, affording the desired compound in 83% yield.
¹H-NMR (300 MHz, CDCl₃ δ TMS): 2.01 (m, 4 H), 3.44 (m, 4 H), 3.58 (s, 2 H), 6.36 (d, J = 8.7 Hz,1 H), 7.40 (d, J = 8.7 Hz, 1 H), 8.04 (s, 1 H).

### c) Title compound

A mixture of 0.3 g (1.6 mmol) of the compound obtained in the preceding section, 1.6 mL of concentrated HCI and 1.6 mL of H₂O was heated at reflux for 18 h. The resulting mixture was concentrated, H₂O was added and the mixture was basified with 25% NaOH solution to pH 5. The solution was extracted with hot EtOAc and the layers were separated. The organic phase was dried over MgSO₄ and concentrated, affording 92 mg of the desired compound (28% yield).
¹H-NMR (300 MHz, CDCl₃ δ TMS): 2.03 (m, 4 H), 3.46 (s, 2 H), 3.49 (m, 4 H), 6.44 (d, J = 8.7 Hz, 1 H), 7.56 (d, J = 8.7 HZ, 1 H), 8.01 (s, 1 H), 9.92 (s, 1 H).

### Example 1

### 4-Chloro-1-(4-methylsulfonylphenyl)-5-[4-(1-pyrrolidinyl)phenyl]imidazole

0.30 g (0.85 mmol) of 4-chloro-5-(4-fluorophenyl)-1-(4-methylsulfonylphenyl)imidazole (obtained in reference example 2), 10 mL of pyrrolidine and 10 mL of THF were introduced into a reaction vessel and the mixture was heated at 150 °C overnight. The mixture was then allowed to cool to room temperature and was concentrated. The residue was treated with a mixture of CHCl₃ and H₂O, the layers were separated and the organic phase was dried over MgSO₄ and concentrated. A crude product was obtained, which was purified by chromatography on silica gel, using EtOAc/hexane mixtures of increasing polarity as eluent, to give 0.27 g of the title compound of the example as a yellow solid (72% yield).
¹H-NMR (300 MHz, CDCl₃ δ TMS): 2.11 (t, J = 6.6 Hz, 4 H), 3.17 (s, 3H), 3.38 (t, J = 6.6 Hz, 4 H), 6.58 (d, J = 8.7 Hz, 2 H), 7.09 (d, J = 8.7 Hz, 2 H), 7.43 (d, J = 8.7 Hz, 2 H), 7.70 (s, 1 H), 8.04 (d, J = 8.7 Hz, 2 H).

### Example 2

### 1-(4-Methylsulfonylphenyl)-5-[4-(1-pyrrolidinyl)phenyl]imidazole

Following a similar procedure to that described in example 1, but starting from 5-(4-fluorophenyl)-1-(4-methylsulfonylphenyl)imidazole (obtained in reference example 1) and increasing the reaction time to two days, the title compound of the example was obtained as a yellow solid (76% yield).
¹H-NMR (300 MHz, CDCl₃ δ TMS): 2.07 (t, J = 6.6 Hz, 4 H), 3.15 (s, 3 H), 3.34 (t, J = 6.6 Hz, 4 H), 6.52 (d, J = 8.7 Hz, 2 H), 7.02 (d, J = 8.7 Hz, 2 H), 7.21 (s, 1 H), 7.45 (d, J = 8.7 Hz, 2 H), 7.75 (s, 1 H), 8.01 (d, J = 8.7 Hz, 2 H).

### Example 3

### 4-Chloro-5-[4-(3-hydroxypyrrolidin-1-yl)phenyl]-1-(4-methylsulfonylphenyl)imidazole

Following a similar procedure to that described in example 1, but using 3-hydroxypyrrolidine instead of pyrrolidine and in the absence of THF, the title compound of the example was obtained as a white solid (42% yield).
¹H-NMR (300 MHz, CD₃OD δ TMS): 2.05 (m, 2 H), 3.07 (s, 3 H), 3.25 (m, 2 H), 3.44 (m, 2 H), 4.50 (s, 1 H), 6.46 (d, J = 8.7 Hz, 2 H), 6.97 (d, J = 8.7 Hz, 2 H), 7.34 (d, J = 8.7 Hz, 2 H), 7.37 (s, 1 H), 7.66 (s, 1 H), 7.92 (d, J = 8.7 Hz, 2 H).

### Example 4

### 4-Chloro-5-[4-(2-methylpyrrolidin-1-yl)phenyl]-1-(4-methylsulfonylphenyl)imidazole

Following a similar procedure to that described in example 1, but using 2-methylpyrrolidine instead of pyrrolidine and increasing the reaction time to 8 days, the title compound of the example was obtained as a yellow solid (20% yield).
¹H-NMR (300 MHz, CDCl₃ δ TMS): 1.17 (d, J = 6.3 Hz, 3 H), 1.73 (m, 1 H), 2.03 (m, 3 H), 3.08 (s, 3 H), 3.17 (m, 1 H), 3.41 (m, 1 H), 3.88 (m, 1 H), 6.51 (d, J = 9 Hz, 2 H), 7.00 (d, J = 9 Hz, 2 H), 7.35 (d, J = 8.7 Hz, 2 H), 7.61 (s, 1 H), 7.95 (d, J = 8.7 Hz, 2 H).

### Example 5

### 4-[4-Chloro-5-[4-(1-pyrrolidinyl)phenyl]imidazol-1-yl]benzenesulfonamide, hydrochloride

0.21 g (0.60 mmol) of 4-[4-chloro-5-(4-fluorophenyl)imidazol-1-yl]benzenesulfonamide (obtained in reference example 5), 10 mL of pyrrolidine and 10 mL of THF were introduced into a reaction vessel and the mixture was heated at 150 °C overnight. The mixture was then allowed to cool to room temperature and was concentrated. The residue was treated with a mixture of CHCl₃ and H₂O, and the aqueous phase was brought to pH 6 by the addition of 1 N HCI solution. The layers were separated, the aqueous phase was extracted with CHCl₃, and the combined organic phases were dried over MgSO₄, filtered, and concentrated. The crude product obtained was purified by chromatography on silica gel, using MeOH/EtOAc/hexane mixtures of increasing polarity as eluent, affording 57 mg of the title compound of the example as a white solid (22% yield).
¹H-NMR (300 MHz, DMSO δ TMS): 1.92 (m, 4 H), 3.19 (m, 4H), 6.48 (d, J = 8.7 Hz, 2 H), 6.97 (d, J = 8.7 Hz, 2 H), 7.41 (d, J = 8.7 Hz, 2 H), 7.45 (s, 2 H), 7.82 (d, J = 8.7 Hz, 2 H), 7.99 (s, 1 H).

### Example 6

### 4-Chloro-5-[3-chloro-4-(1-pyrrolidinyl)phenyl]-1-(4-methylsulfonylphenyl)imidazole

A mixture of 0.120 g (0.298 mmol) of 4-chloro-1-(4-methylsulfonylphenyl)-5-[4-(1-pyrrolidinyl)phenyl]imidazole (obtained in example 1), 1 mL of acetonitrile and 0.04 g (0.298 mmol) of N-chlorosuccinimide was heated at reflux for 4 h. The resulting mixture was concentrated, EtOAc was added and it was washed with 1N NaOH solution. The organic phase was dried over MgSO₄ and concentrated, affording a crude product that was purified by chromatography on silica gel using EtOAc/hexane mixtures of increasing polarity as eluent. 0.103 g of the title compound of the example was obtained as a creamy solid (79% yield).
¹H-NMR (300 MHz, CDCl₃ δ TMS): 1.95 (m, 4 H), 3.08 (s, 3 H), 3.45 (m, 4 H), 6.72, (d, J = 8.7 Hz, 1 H), 6.84 (d, J = 8.7 Hz, 1 H), 7.14 (s, 1 H), 7.34 (d, J = 8.7 Hz, 2 H), 7.61 (s, 1 H), 7.98 (d, J = 8.7 Hz, 2 H).

### Example 7

### 4-Chloro-1-(4-methylsulfonylphenyl)-5-[4-(2,5-dioxopyrrolidin-1-yl)phenyl]imidazole

0.3 g (0.86 mmol) of 5-(4-aminophenyl)-4-chloro-1-(4-methylsulfonylphenyl)imidazole (obtained in reference example 3), 0.086 g of succinic anhydride and 10.6 mL of toluene were placed in a flask under argon and the mixture was refluxed for 1 h. 0.23 mL of Et₃N was added and the mixture was further refluxed for 4 days. The mixture was then concentrated and the crude product obtained was purified by chromatography on silica gel using EtOAc/hexane mixtures of increasing polarity as eluent. 58 mg of the title compound of the example was obtained as a white solid (16% yield).
¹H-NMR (300 MHz, CDCl₃ δ TMS): 2.91 (s, 4 H), 3.10 (s, 3 H), 7.29 - 7.37 (m, 6 H), 7.68 (s, 1 H), 7.99 (d, J = 8.4 Hz, 2 H).

### Example 8

### 4-Chloro-1-(4-methylsulfonylphenyl)-5-[4-(2-oxo-3-pyrrolin-1-yl)phenyl]imidazole

0.3 g (0.86 mmol) of 5-(4-aminophenyl)-4-chloro-1-(4-methylsulfonylphenyl)imidazole (obtained in reference example 3), 0.112 g (0.86 mmol) of 2,5-dimethoxy-2,5-dihydrofuran, 0.2 mL of 10% HCI solution, 1 mL of H₂O and 2 mL of THF were placed in a flask and the mixture was stirred at room temperature for 3 h. The mixture was then concentrated, and the residue was partitioned between CHCl₃ and 1N NaOH solution. The layers were separated and the organic phase was dried over MgSO₄. The solvent was removed and the resulting crude product was purified by chromatography on silica gel using EtOAc/hexane mixtures of increasing polarity as eluent, to give 79 mg of the title compound of the example as a white solid (22% yield).
¹H-NMR (300 MHz, CDCl₃ δ TMS): 3.10 (s, 3 H), 4.46 (s, 2 H), 6.29 (d, J = 6 Hz, 1 H), 7.22 (m, 3 H), 7.34 (d, J = 8.7 Hz, 2 H), 7.66 (s, 1 H), 7.77 (d, J = 8.7 Hz, 2 H), 7.97 (d, J = 8.7 Hz, 2 H).

### Example 9

### 4-Chloro-1-(4-methylsulfonylphenyl)-5-[4-(2-oxooxazolidin-3-yl)phenyl]imidazole

### a) 4-Chloro-5-[4-(2-chloroethoxycarbonylamino)phenyl]-1-(4-methylsulfonylphenyl)imidazole

To a mixture of 0.3 g (0.86 mmol) of 5-(4-aminophenyl)-4-chloro-1-(4-methylsulfonylphenyl)imidazole (obtained in reference example 3), 0.69 mL of DMF and 0.07 mL of pyridine, 0.088 mL (0.86 mmol) of 2-chloroethyl chloroformate dissolved in 0.215 mL of Et₂O was added dropwise and under argon. The resulting mixture was stirred at room temperature for 3 h, was then concentrated and the residue was treated with a mixture of EtOAc and H₂O. The layers were separated, the aqueous phase was extracted with EtOAc and the combined organic phases were dried over MgSO₄ and concentrated. 0.43 g of a crude product was obtained, which was directly used in the following step.
¹H-NMR (300 MHz, CDCl₃ δ TMS): 2.95 (s, 3 H), 3.72 (t, J = 5.4 Hz, 2 H), 4.41 (t, J = 5.4 Hz, 2 H), 7.13 (d, J = 8.7 Hz, 2 H), 7.31 (d, J = 8.7 Hz, 2 H), 7.43 (d, J = 8.4 Hz, 2 H), 7.65 (s, 1 H), 7.95 (d, J = 8.4 Hz, 2 H), 8.01 (s, 1 H).

### b) Title compound

To a mixture of 0.43 g (0.86 mmol) of the crude product obtained in the preceding section, 19.7 mL of 70% EtOH and 1.4 mL of THF, 0.057 g (1.032 mmol) of KOH dissolved in 2.77 mL of 70% EtOH was added and the resulting mixture was stirred at room temperature overnight. The mixture was then concentrated, and the residue was partitioned between H₂O and CHCl₃. The layers were separated and the aqueous phase was extracted with CHCl₃. The combined organic phases were dried over MgSO₄ and concentrated. The crude product was purified by chromatography on silica gel using EtOAc/hexane mixtures of increasing polarity as eluent, to give 230 mg of the title compound of the example as a white solid (64% yield).
¹H-NMR (300 MHz, CDCl₃ δ TMS): 3.10 (s, 3 H), 4,07 (t, J = 8.4 Hz, 2 H), 4.51 (t, J = 8.4 Hz, 2 H), 7.22 (d, J = 8.7 Hz, 2 H), 7.32 (d, J = 8.7 Hz, 2 H), 7.56 (d, J = 8.7 Hz, 2 H), 7.66 (s, 1 H), 7.97 (d, J = 8.7 Hz, 2 H).

### Example 10

### 4-Chloro-1-(4-methylsulfonylphenyl)-5-[4-(2-oxopyrrolidin-1-yl)phenyl]imidazole

0.3 g (0.86 mmol) of 5-(4-aminophenyl)-4-chloro-1-(4-methylsulfonylphenyl)imidazole (obtained in reference example 3), 0.12 mL (0.885 mmol) of Et₃N and 2.5 mL of THF were placed in a flask, and the mixture was stirred at room temperature under argon. Next 0.124 g (0.885 mmol) of 4-chlorobutyryl chloride was slowly added and the resulting mixture was stirred at room temperature for 2 h. The mixture was then cooled to 0 °C in an ice bath and 0.224 g (2 mmol) of potassium *tert*-butoxide in 2 mL of THF was added dropwise. The mixture was stirred for 1 h at this temperature, was then allowed to warm up to room temperature and was concentrated. The residue was treated with CHCl₃ and H₂O, the phases were separated and the organic phase was dried over MgSO₄ and concentrated. The crude product obtained was purified by chromatography on silica gel using EtOAc/hexane mixtures of increasing polarity as eluent, affording 225 mg of the title compound of the example as a white solid (63% yield).
¹H-NMR (300 MHz, CDCl₃ δ TMS): 2.18 (m, 2 H), 2.63 (t, J = 7.8 Hz, 2 H), 3.09 (s, 3 H), 3.87 (t, J = 7.2 Hz, 2 H), 7.20 (d, J = 8.7 Hz, 2 H), 7.33 (d, J = 8.7 Hz, 2 H), 7.66 (m, 3 H), 7.96 (d, J = 8.7 Hz, 2 H).

### Example 11

### 1-(4-Methylsulfonylphenyl)-5-[6-(1-pyrrolidinyl)-3-pyridyl]imidazole

Following a similar procedure to that described in section b of reference example 8, but starting from 5-(6-chloro-3-pyridyl)-1-(4-methylsulfonylphenyl)imidazole (obtained in reference example 4), the title compound of the example was obtained as a white solid and in quantitative yield.
¹H-NMR (300 MHz, CDCl₃ δ TMS): 2.03 (m, 4 H), 3.10 (s, 3 H), 3.44 (m, 4 H), 6.29 (d, J = 8.7 Hz, 1 H), 7.13 (d, J = 8.7 Hz, 1 H), 7.26, (s, 1 H), 7.40 (d, J = 8.7 Hz, 2 H), 7.73 (s, 1 H), 7.97 (d, J = 8.7 Hz, 2 H), 7.99 (s, 1 H).

### Example 12

### 4-Chloro-1-(4-methylsulfonylphenyl)-5-[6-(1-pyrrolidinyl)-3-pyridyl]imidazole

Following a similar procedure to that described in example 6, but starting from 1-(4-methylsulfonylphenyl)-5-[6-(1-pyrrolidinyl)-3-pyridyl]imidazole (obtained in example 11) and allowing the reaction to proceed overnight, the title compound of the example was obtained as a yellow solid (13% yield).
¹H-NMR (300 MHz, CDCl₃ δ TMS): 2.03 (m, 4 H), 3.10 (s, 3 H), 3.47 (s, broad signal, 4 H), 6.34 (d, J = 9 Hz, 1 H), 7.24 (d, J = 8.7 Hz, 1 H), 7.38 (d, J = 6.6 Hz; 2 H), 7.64 (s, 1 H), 7.97 (d, J = 6.6 Hz, 2 H), 7.99 (s, 1 H).

### Example 13

### 3-[4-(2,5-Dioxopyrrolidin-1-yl)phenyl]-4-(4-methylsulfonylphenyl)-5H-furan-2-one

Following a similar procedure to that described in example 7, but starting from 3-(4-aminophenyl)-4-(4-methylsulfonylphenyl)-*5H*-furan-2-one (obtained in reference example 6), and allowing the reaction to proceed overnight, the title compound of the example was obtained as a white solid (60% yield).
¹H-NMR (300 MHz, CDCl₃ δ TMS): 2.92 (s, 4 H), 5.20 (s, 2 H), 7.38 (d, J = 8.7 Hz, 2 H), 7.54 (d, J = 8.7 HZ, 2 H), 7.54 (d, J = 8.7 Hz, 2 H), 7.96 (d, J = 8.7 Hz, 2 H).

### Example 14

### 4-(4-Methylsulfonylphenyl)-3-[4-(2-oxo-3-pyrrolin-1-yl)phenyl]-55H-furan-2-one

Following a similar procedure to that described in example 8, but starting from 3-(4-aminophenyl)-4-(4-methylsulfonylphenyl)-*5H*-furan-2-one (obtained in reference example 6), and increasing the reaction time to one night, the title compound of the example was obtained as a white solid (13% yield).
¹H-NMR (300 MHz, CDCl₃ δ TMS): 3.10 (s, 3 H), 4.48 (m, 2 H), 5.18 (s, 2 H), 6.30 (d, J = 6 Hz, 1 H), 7.22 (d, J = 6 Hz, 1 H), 7.46 (d, J = 8.7 Hz, 2 H), 7.55 (d, J = 8.7 Hz, 2 H), 7.80 (d, J = 8.7 Hz, 2 H), 7.94 (d, J = 8.7 Hz, 2 H).

### Example 15

### 4-(4-Methylsulfonylphenyl)-3-[4-(1-pyrrolidinyl)phenyl]-5H-furan-2-one

A mixture of 1 g (3 mmol) of 3-(4-aminophenyl)-4-(4-methylsulfonylphenyl)-*5H*-furan-2-one (obtained in reference example 6), 0.36 mL (3 mmol) of 1,4-dibromobutane, 0.41 mL of Et₃N and 10 mL of acetonitrile was refluxed overnight. The resulting mixture was concentrated and the crude product obtained was purified by chromatography on silica gel using EtOAc/hexane mixtures of increasing polarity as eluent. 0.316 g of the title compound of the example was obtained as a yellow solid (27% yield).
¹H-NMR (300 MHz, CDCl₃ δ TMS): 1.96 (m, 4 H), 3.01 (s, 3 H), 3.25 (m, 4 H), 5.05 (s, 2 H), 6.47 (d, J = 9 Hz, 2 H), 7.25 (d, J = 9 Hz, 2 H), 7.52 (d, J = 8.4 Hz, 2 H), 7.85 (d, J = 8.4 Hz, 2 H).

### Example 16

### 3-[3-Chloro-4-(1-pyrrolidinyl)phenyl]-4-(4-methylsulfonylphenyl)-5H-furan-2-one

Following a similar procedure to that described in example 6, but starting from 4-(4-methylsulfonylphenyl)-3-[4-(1-pyrrolidinyl)phenyl]-*5H*-furan-2-one (obtained in example 15), the title compound of the example was obtained as a yellow solid (73% yield).
¹H-NMR (300 MHz, CDCl₃ δ TMS): 1.97 (m, 4 H), 3.09 (s, 3 H), 3.48 (m, 4 H), 5.14 (s, 2 H), 6.77 (d, J = 8.7 Hz, 1 H), 7.16 (d, J = 8.7 Hz, 1 H), 7.41 (s, 1 H), 7.58 (d, J = 8.7 Hz, 2 H), 7.96 (d, J = 8.7 Hz, 2 H).

### Example 17

### 4-(4-Methylsulfonylphenyl)-3-[6-(1-pyrrolidinyl)-3-pyridyl]-5H-furan-2-one

Following a similar procedure to that described in section b of reference example 6, but using 6-(1-pyrrolidinyl)-3-pyridylacetic acid (obtained in reference example 8) instead of 4-nitrophenylacetic acid, the title compound of the example was obtained as a yellow solid (13% yield).
¹H-NMR (300 MHz, CDCl₃ δ TMS): 2.03 (m, 4 H), 3.09 (s, 3 H), 3.48 (m, 4 H), 5.13 (s, 2 H), 6.40 (d, J = 8.7 Hz, 1 H), 7.60 (d, J = 8.4 Hz, 2 H), 7.65 (d, J = 8.7 Hz, 1 H), 7.95 (d, J = 8.4 Hz, 2 H), 8.17 (s, 1 H).

### Example 18

### 4-[5-[4-(2-Oxo-3-pyrrolin-1-yl)phenyl]-3-trifluoromethyl-1H-pyrazol-1-yl]benzenesulfonamide

Following a similar procedure to that described in example 8, but starting from 4-[5-(4-aminophenyl)-3-trifluoromethyl-*1H*-pyrazol-1-yl]benzenesulfonamide (obtained in reference example 7), and increasing the reaction time to one night, the title compound of the example was obtained as a white solid (13% yield).
¹H-NMR (300 MHz, CDCl₃ δ TMS): 4.48 (m, 2 H), 5.06 (s, 2 H), 6.29 (d, J = 6 Hz, 1 H), 6.77 (s, 1 H), 7.24 (d, J = 6 Hz, 1 H), 7.24 (d, J = 8.4 Hz, 2 H), 7.48 (d, J = 8.4 Hz, 2 H), 7.78 (d, J = 8.7 Hz, 2 H), 7.91 (d, J = 8.7 Hz, 2H).

### Example 19

### 4-[5-[4-(1-Pyrrolidinyl)phenyl]-3-trifluoromethyl-1H-pyrazol-1-yl]benzenesulfonamide

Following a similar procedure to that described in example 15, but starting from 4-[5-(4-aminophenyl)-3-trifluoromethyl-*1H*-pyrazol-1-yl]benzenesulfonamide (obtained in reference example 7), the title compound of the example was obtained as a yellow solid (7% yield).
¹H-NMR (300 MHz, CDCl₃ δ TMS): 2.00 (m, 4 H), 3.26 (m, 4 H), 5.23 (s, 2 H), 6.46 (d, J = 8.7 Hz, 2 H), 6.65 (s, 1 H), 7.03 (d, J = 8.7 Hz, 2 H), 7.48 (d, J = 8.7 Hz, 2 H), 7.87 (d, J = 8.7 Hz, 2 H).

## Claims

1. A compound of general formula **I**: 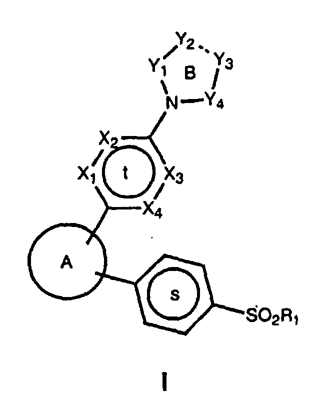 wherein:
A represents an unsaturated or partially insaturated 5-membered ring, which can optionally contain from 1 to 3 heteroatoms selected from N, O and S, wherein the two aromatic substituents s and t are placed on adjacent atoms of ring A, and wherein A can be optionally substituted with one or more substituents R₂;
R₁ represents C₁₋₈ alkyl, C₁₋₈ haloalkyl or -NR₃R₄;
R₂ represents C₁₋₄ alkyl, C₁₋₄ haloalkyl, halogen, oxo, cyano, nitro, -CHO, -COCH₃ or -COOR₃;
R₃ represents hydrogen, C₁₋₈ alkyl or arylC₀₋₈ alkyl;
R₄ represents hydrogen, C₁₋₈ alkyl, arylC₁₋₈ alkyl, -COR₅ or -COOR₅;
R₅ represents C₁₋₈ alkyl or C₁₋₈ haloalkyl;
X₁, X₂, X₃ and X₄ represent all CR₆, or one, two or three of X₁, X₂, X₃ and X₄ represent nitrogen and the other groups X₁, X₂, X₃ and X₄ represent CR₆;
each R₆ independently represents hydrogen, halogen, C₁₋₃ alkyl or C₁₋₃ alkoxy;
the dotted line in ring B represents a single or double bond;
Y₁ and Y₄ independently represent CR₇R₇ or CO;
when the dotted line represents a double bond Y₂ and Y₃ represent CR₈;
when the dotted line represents a single bond Y₂ and Y₃ represent CR₈R₈, and additionally Y₂ can represent CO if Y₁ is different from CO, and additionally Y₃ can represent CO if Y₄ is different from CO or Y₃ can represent NR₉, O or S if Y₄ represents CO;
each R₇ independently represents hydrogen, methyl or ethyl;
each R₈ independently represents hydrogen, methyl, ethyl, hydroxy, methoxy or halogen;
R₉ represents hydrogen or C₁₋₄ alkyl;
aryl in the above definitions represent phenyl or naphthyl, which can be optionally substituted with one or more groups selected from C₁₋₈ alkyl, halogen, C₁₋₈ haloalkyl, cyano, nitro, R₁₀OC₀₋₈ alkyl, R₁₀SC₀₋₈ alkyl, -NR₁₀R₁₁, -NR₁₀COR₁₁, -COR₁₀ or -COOR₁₀;
R₁₀ represents hydrogen, C₁₋₈ alkyl or benzyl;
R₁₁ represents C₁₋₈ alkyl or C₁₋₈ haloalkyl;
and the salts and solvates thereof.

2. A compound according to claim 1 wherein R₁ represents methyl or amino.

3. A compound according to claim 1 or 2 wherein X₁, X₂, X₃ and X₄ represent all CR₆ or one of X₂ or X₃ represents N and the others represent CR₆.

4. A compound according to any of claims 1 to 3 wherein A represents imidazole, pyrazole, furanone, thiophene, isoxazole, oxazole, oxazolone or cyclopentene, wherein A can be optionally substituted with one or more substituents R₂.

5. A compound according to claim 4 wherein A represents imidazole, furanone or pyrazole, wherein A can be optionally substituted with one or more substituents R₂.

6. A compound according to any of claims 1 to 5 wherein R₇ and R₈ represent hydrogen.

7. A compound according to any of claims 1 to 5 wherein Y₁, Y₂, Y₃ and Y₄ represent CH₂ and the dotted line represents a single bond, or one of Y₁ or Y₄ represents CO and the other represents CH₂ and Y₂ and Y₃ represent CH₂ in which case the dotted line represents a single bond or Y₂ and Y₃ represent CH in which case the dotted line represents a double bond.

8. A compound according to claim 1 selected from:
4-chloro-1-(4-methylsulfonylphenyl)-5-[4-(1-pyrrolidinyl)phenyl]imidazole;
1-(4-methylsulfonylphenyl)-5-[4-(1-pyrrolidinyl)phenyl]imidazole;
4-chloro-5-[4-(3-hydroxypyrrolidin-1-yl)phenyl]-1-(4-methylsulfonylphenyl)imidazole;
4-chloro-5-[4-(2-methylpyrrolidin-1-yl)phenyl]-1-(4-methylsulfonylphenyl)imidazole;
4-[4-chloro-5-[4-(1-pyrrolidinyl)phenyl]imidazol-1-yl]benzenesulfonamide;
4-chloro-5-[3-chloro-4-(1-pyrrolidinyl)phenyl]-1-(4-methylsulfonylphenyl)imidazole;
4-chloro-1-(4-methylsulfonylphenyl)-5-[4-(2,5-dioxopyrrolidin-1-yl)phenyl]imidazole;
4-chloro-1-(4-methylsulfonylphenyl)-5-[4-(2-oxo-3-pyrrolin-1-yl)phenyl]imidazole;
4-chloro-1-(4-methylsulfonylphenyl)-5-[4-(2-oxooxazolidin-3-yl)phenyl]imidazole;
4-chloro-1-(4-methylsulfonylphenyl)-5-[4-(2-oxopyrrolidin-1-yl)phenyl]imidazol;
1-(4-methylsulfonylphenyl)-5-[6-(1-pyrrolidinyl)-3-pyridyl]imidazole;
4-chloro-1-(4-methylsulfonylphenyl)-5-[6-(1-pyrrolidinyl)-3-pyridyl]imidazole;
3-[4-(2,5-dioxopyrrolidin-1-yl)phenyl]-4-(4-methylsulfonylphenyl)-*5H*-furan-2-one;
4-(4-methylsulfonylphenyl)-3-[4-(2-oxo-3-pyrrolin-1-yl)phenyl]-*5H*-furan-2-one;
4-(4-methylsulfonylphenyl)-3-[4-(1-pyrrolidinyl)phenyl]-*5H*-furan-2-one;
3-[3-chloro-4-(1-pyrrolidinyl)phenyl]-4-(4-methylsulfonylphenyl)-*5H*-furan-2-one;
4-(4-methylsulfonylphenyl)-3-[6-(1-pyrrolidinyl)-3-pyridyl]-*5H*-furan-2-one;
4-[5-[4-(2-oxo-3-pyrrolin-1-yl)phenyl]-3-trifluoromethyl-*1H*-pyrazol-1-yl]benzenesulfonamide;
4-[5-[4-(1-pyrrolidinyl)phenyl]-3-trifluoromethyl-*1H*-pyrazol*-*1-yl]benzenesulfonamide;
or a salt or solvate thereof.

9. Process for preparing a compound of formula **I** according to claim 1, which comprises:
(a) when in a compound of formula **I** Y₁ and Y₄ represent CR₇R₇, reacting a compound of formula **II** 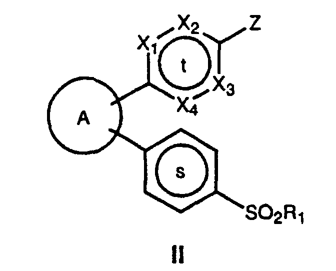 wherein X₁, X₂, X₃, X₄, R₁ and A have the meaning described in claim 1 and Z represents fluoro or, when one of X₂ or X₃ represents nitrogen, Z represents chloro, with an amine of formula **IV** 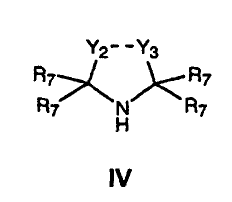 wherein R₇, Y₂, Y₃ and the dotted line have the meaning described in claim 1; or
(b) when in a compound of formula **I** Y₁ and Y₄ represent CR₇R₇, reacting a compound of formula **III** 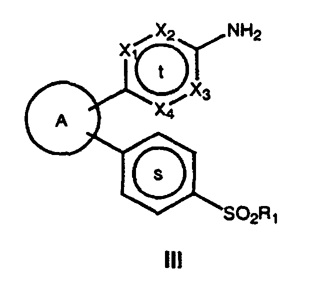 wherein X₁, X₂, X₃, X₄, R₁ and A have the meaning described in claim 1, with a compound of formula **V** 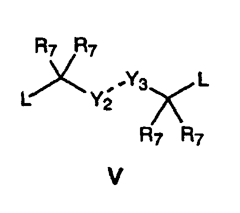 wherein R₇, Y₂, Y₃ and the dotted line have the meaning described in claim 1 and L represents a good leaving group; or
(c) when in a compound of formula **I** Y₁ and/or Y₄ represents CO, reacting a compound of formula **III** with a compound of formula **VI** or **VI'** respectively 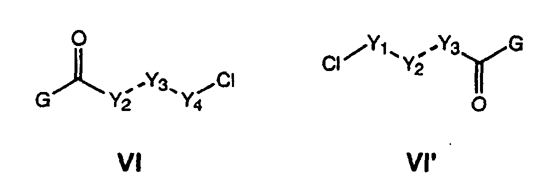 wherein Y₁, Y₂, Y₃, Y₄ and the dotted line have the meaning described in claim 1 and G represents a good leaving group; or
(d) when in a compound of formula **I** Y₄ represents CO and Y₃ represents NH, reacting a compound of formula **III** with an isocyanate of formula **VII** 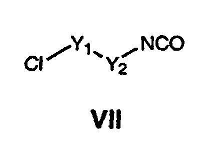 wherein Y₁ and Y₂ have the meaning described in claim 1; or
(e) when in a compound of formula **I** Y₁ and Y₄ represent CO, reacting a compound of formula **III** with a compound of formula **VIII** 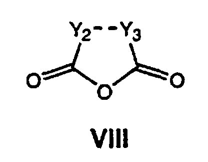 wherein Y₂, Y₃ and the dotted line have the meaning described in claim 1; or
(f) when in a compound of formula **I** one of Y₁ or Y₄ represents CH₂ and the other represents CO, and the dotted line represents a double bond, reacting a compound of formula **III** with a compound of formula **IX** 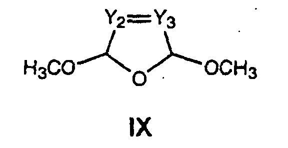 wherein Y₂ and Y₃ have the meaning described in claim 1; or
(g) converting, in one or a plurality of steps, a compound of formula **I** into another compound of formula **I;** and
(h) if desired, after the above steps, reacting a compound of formula **I** with an acid or a base to give the corresponding salt.

10. A pharmaceutical composition which comprises an effective amount of a compound of formula **I** according to any of claims 1 to 8 or a pharmaceutically acceptable salt or solvate thereof and one or more pharmaceutically acceptable excipients.

11. Use of a compound of formula I according to any of claims 1 to 8 or a pharmaceutically acceptable salt or solvate thereof for the manufacture of a medicament for the treatment or prevention of diseases mediated by cyclooxygenase.

12. Use of a compound of formula **I** according to any of claims 1 to 8 or a pharmaceutically acceptable salt or solvate thereof for the manufacture of a medicament for the treatment or prevention of diseases mediated by cyclooxygenase-2.

13. Use of a compound of formula **I** according to any of claims 1 to 8 or a pharmaceutically acceptable salt or solvate thereof for the manufacture of a medicament for the treatment of inflammation, pain and/or fever.

14. Use of a compound of formula **I** according to any of claims 1 to 8 or a pharmaceutically acceptable salt or solvate thereof for the manufacture of a medicament for inhibiting prostanoid-induced smooth muscle contraction.

15. Use of a compound of formula **I** according to any of claims 1 to 8 or a pharmaceutically acceptable salt or solvate thereof for the manufacture of a medicament for the treatment or prevention of dysmenorrhea, preterm labour, asthma and bronchitis.

16. Use of a compound of formula **I** according to any of claims 1 to 8 or a pharmaceutically acceptable salt or solvate thereof for the manufacture of a medicament for the treatment or prevention of familial adenomatous polyposis.

17. Use of a compound of formula **I** according to any of claims 1 to 8 or a pharmaceutically acceptable salt or solvate thereof for the manufacture of a medicament for the treatment or prevention of cancer.

18. Use according to claim 17 wherein the cancer is a gastrointestinal cancer.

19. Use according to claim 18 wherein the gastrointestinal cancer is colon cancer.

20. Use of a compound of formula **I** according to any of claims 1 to 8 or a pharmaceutically acceptable salt or solvate thereof for the manufacture of a medicament for the treatment or prevention of cerebral infarction, epilepsy, and neurodegenerative diseases such as Alzheimer's disease and dementia.

## Patentansprüche

1. Verbindung der allgemeinen Formel I: 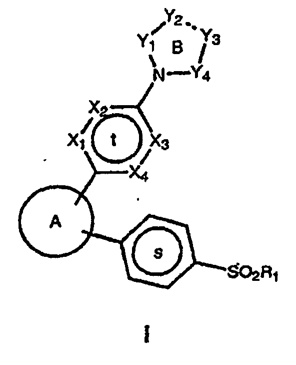 worin
A einen ungesättigten oder teilweise ungesättigten 5-gliedrigen Ring bedeutet, der gegebenenfalls 1 bis 3 Heteroatome, ausgewählt unter N, O und S, enthalten kann, worin die zwei aromatischen Substituenten s und t an benachbarten Atomen des Rings A vorliegen, und worin A gegebenenfalls mit einem oder mehreren Substituenten R₂ substituiert sein kann; R₁ C₁₋₈-Alkyl, C₁₋₈-Haloalkyl oder -NR₃R₄ bedeutet;
R₂ C₁₋₄-Alkyl, C₁₋₄-Haloalkyl, Halogen, Oxo, Cyano, Nitro, -CHO, -COCH₃ oder -COOR₃ bedeutet;
R₃ Wasserstoff, C₁₋₈-Alkyl oder Aryl-C₀₋₈-alkyl bedeutet;
R₄ Wasserstoff, C₁₋₈-Alkyl, Aryl-C₁₋₈-alkyl, -COR₅ oder -COOR₅ bedeutet;
R₅ C₁₋₈-Alkyl oder C₁₋₈-Haloalkyl bedeutet;
X₁, X₂, X₃ und X₄ sämtlich CR₆ bedeuten, oder eines, zwei oder drei von X₁, X₂, X₃ und
X₄ Stickstoff und die anderen Gruppen X₁, X₂, X₃ und X₄ CR₆ bedeuten;
jedes R₆ unabhängig Wasserstoff, Halogen, C₁₋₃-Alkyl oder C₁₋₃-Alkoxy bedeutet;
die gestrichelte Linie des Rings B eine Einfach- oder Doppelbindung bedeutet;
Y₁ und Y₄ unabhängig CR₇R₇ oder CO bedeuten;
wenn die gestrichelte Linie eine Doppelbindung bedeutet, Y₂ und Y₃ CR₈ bedeuten;
wenn die gestrichelte Linie eine Einfachbindung bedeutet, Y₂ und Y₃ CR₈R₈ bedeuten, und
zusätzlich Y₂ CO bedeuten kann, wenn Y₁ von CO verschieden ist, und zusätzlich Y₃ CO bedeuten kann, wenn Y₄ von CO verschieden ist, oder Y₃ NR₉, O oder S bedeuten kann, wenn Y₄ CO bedeutet;
jedes R₇ unabhängig Wasserstoff, Methyl oder Ethyl bedeutet;
jedes R₈ unabhängig Wasserstoff, Methyl, Ethyl, Hydroxy, Methoxy oder Halogen bedeutet;
R₉ Wasserstoff oder C₁₋₄-Alkyl bedeutet;
Aryl in den vorstehenden Definitionen Phenyl oder Naphthyl bedeutet, die gegebenenfalls mit einer oder mehreren Gruppen substituiert sein können, ausgewählt unter C₁₋₈-Alkyl, Halogen, C₁₋₈-Haloalkyl, Cyano, Nitro, R₁₀OC₀₋₈-alkyl, R₁₀SC₀₋₈-alkyl, -NR₁₀R₁₁, -NR₁₀COR₁₁, -COR₁₀ oder -COOR₁₀;
R₁₀ Wasserstoff, C₁₋₈-Alkyl oder Benzyl bedeutet;
R₁₁ C₁₋₈-Alkyl oder C₁₋₈-Haloalkyl bedeutet;
und die Salze und Solvate hiervon.

2. Verbindung gemäß Anspruch 1, worin R₁ Methyl oder Amino bedeutet.

3. Verbindung gemäß Anspruch 1 oder 2, worin X₁, X₂, X₃ und X₄ sämtlich CR₆ bedeuten oder eines von X₂ oder X₃ N bedeutet und die anderen CR₆ bedeuten.

4. Verbindung gemäß einem der Ansprüche 1 bis 3, worin A Imidazol, Pyrazol, Furanon, Thiophen, Isoxazol, Oxazol, Oxazolon oder Cyclopenten bedeutet, worin A gegebenenfalls mit einem oder mehreren Substituenten R₂ substituiert sein kann.

5. Verbindung gemäß Anspruch 4, worin A Imidazol, Furanon oder Pyrazol bedeutet, worin A gegebenenfalls mit einem oder mehreren Substituenten R₂ substituiert sein kann.

6. Verbindung gemäß einem der Ansprüche 1 bis 5, worin R₇ und R₈ Wasserstoff bedeuten.

7. Verbindung gemäß einem der Ansprüche 1 bis 5, worin Y₁, Y₂, Y₃ und Y₄ CH₂ bedeuten und die gestrichelte Linie eine Einfachbindung bedeutet, oder eines von Y₁ oder Y₄ CO bedeutet und das andere CH₂ bedeutet und Y₂ und Y₃ CH₂ bedeuten, in welchem Fall die gestrichelte Linie eine Einfachbindung bedeutet, oder Y₂ und Y₃ CH bedeuten, in welchem Fall die gestrichelte Linie eine Doppelbindung bedeutet.

8. Verbindung gemäß Anspruch 1, ausgewählt unter:
4-Chlor-1-(4-methylsulfonylphenyl)-5-[4-(1-pyrrolidinyl)-phenyl]-imidazol;
1-(4-Methylsulfonylphenyl)-5-[4-(1-pyrrolidinyl)-phenyl]-imidazol;
4-Chlor-5-[4-(3-hydroxypyrrolidin-1-yl)-phenyl]-1-(4-methylsulfonylphenyl)-imidazol;
4-Chlor-5-[4-(2-methylpyrrolidin-1-yl)-phenyl]-1-(4-methylsulfonylphenyl)-imidazol;
4-[4-Chlor-5-[4-(1-pyrrolidinyl)-phenyl]-imidazol-1-yl]-benzolsulfonamid;
4-Chlor-5-[3-chlor-4-(1-pyrrolidinyl)-phenyl]-1-(4-methylsulfonylphenyl)-imidazol;
4-Chlor-1-(4-methylsulfonylphenyl)-5-[4-(2,5-dioxopyrrolidin-1-yl)-phenyl]-imidazol;
4-Chlor-1-(4-methylsulfonylphenyl)-5-[4-(2-oxo-3-pyrrolin-1-yl)-phenyl]-imidazol;
4-Chlor-1-(4-methylsulfonylphenyl)-5-[4-(2-oxooxazolidin-3-yl)-phenyl]-imidazol;
4-Chlor-1-(4-methylsulfonylphenyl)-5-[4-(2-oxopyrrolidin-1-yl)-phenyl]-imidazol;
1-(4-Methylsulfonylphenyl)-5-[6-(1-pyrrolidinyl)-3-pyridyl]-imidazol;
4-Chlor-1-(4-methylsulfonylphenyl)-5-[6-(1-pyrrolidinyl)-3-pyridyl]-imidazol;
3-[4-(2,5-Dioxopyrrolidin-1-yl)-phenyl]-4-(4-methylsulfonylphenyl)-5*H*-furan-2-on;
4-(4-Methylsulfonylphenyl)-3-[4-(2-oxo-3-pyrrolin-1-yl)-phenyl]-5*H*-furan-2-on;
4-(4-Methylsulfonylphenyl)-3-[4-(1-pyrrolidinyl)-phenyl]-5*H*-furan-2-on;
3-[3-Chlor-4-(1-pyrrolidinyl)-phenyl]-4-(4-methylsulfonylphenyl)-5*H*-furan-2-on;
4-(4-Methylsulfonylphenyl)-3-[6-(1-pyrrolidinyl)-3-pyridyl]-5*H*-furan-2-on;
4-[5-[4-(2-Oxo-3-pyrrolin-1-yl)-phenyl]-3-trifluormethyl-1*H*-pyrazol-1-yl]-benzolsulfonamid;
4-[5-[4-(1-Pyrrolidinyl)-phenyl]-3-trifluormethyl-1*H*-pyrazol-1-yl]-benzolsulfonamid;
oder ein Salz oder Solvat hiervon.

9. Verfahren zur Herstellung einer Verbindung der Formel I gemäß Anspruch 1, das umfasst:
(a) wenn in einer Verbindung der Formel I Y₁ und Y₄ CR₇R₇ bedeuten, die Umsetzung einer Verbindung der Formel II 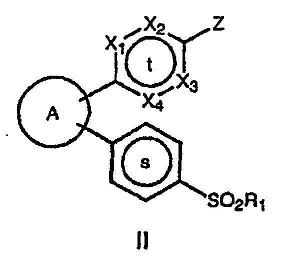 worin X₁, X₂, X₃, X₄, R₁ und A die in Anspruch 1 angegebene Bedeutung besitzen und Z Fluor bedeutet, oder, wenn eines von X₂ oder X₃ Stickstoff bedeutet, Z Chlor bedeutet, mit einem Amin der Formel IV 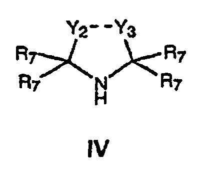 worin R₇, Y₂, Y₃ und die gestrichelte Linie die in Anspruch 1 angegebene Bedeutung besitzen; oder
(b) wenn in einer Verbindung der Formel I Y₁ und Y₄ CR₇R₇ bedeuten, die Umsetzung einer Verbindung der Formel III 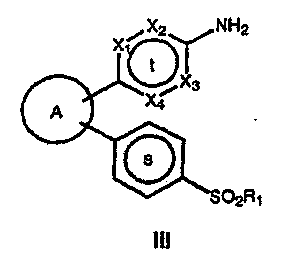 worin X₁, X₂, X₃, X₄, R₁ und A die in Anspruch 1 angegebene Bedeutung besitzen, mit einer Verbindung der Formel V 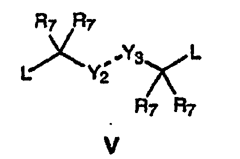 worin R₇, Y₂, Y₃ und die gestrichelte Linie die in Anspruch 1 angegebene Bedeutung besitzen und L eine gute austretende Gruppe wiedergibt; oder
(c) wenn in einer Verbindung der Formel I Y₁ und/oder Y₄ CO bedeutet, die Umsetzung einer Verbindung der Formel III mit einer Verbindung der Formel VI bzw. VI' 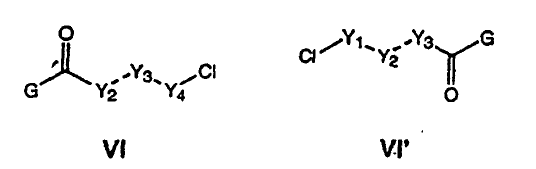 worin Y₁, Y₂, Y₃, Y₄ und die gestrichelte Linie die in Anspruch 1 angegebene Bedeutung besitzen und G für eine gute austretende Gruppe steht; oder
(d) wenn in einer Verbindung der Formel I Y₄ CO bedeutet und Y₃ NH bedeutet, die Umsetzung einer Verbindung der Formel III mit einem Isocyanat der Formel VII 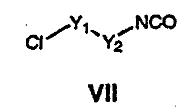 worin Y₁ und Y₂ die in Anspruch 1 angegebene Bedeutung besitzen; oder
(e) wenn in einer Verbindung der Formel I Y₁ und Y₄ CO bedeuten, die Umsetzung einer Verbindung der Formel III mit einer Verbindung der Formel VIII 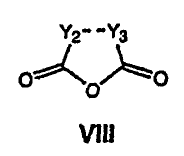 worin Y₂, Y₃ und die gestrichelte Linie die in Anspruch 1 angegebene Bedeutung besitzen; oder
(f) wenn in einer Verbindung der Formel I eines von Y₁ oder Y₄ CH₂ bedeutet und das andere CO bedeutet, und die gestrichelte Linie eine Doppelbindung wiedergibt, die Umsetzung einer Verbindung der Formel III mit einer Verbindung der Formel IX 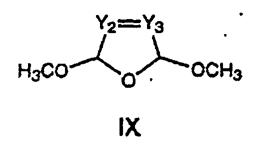 worin Y₂ und Y₃ die in Anspruch 1 angegebene Bedeutung besitzen; oder
(g) die Umwandlung in einer oder mehreren Stufen einer Verbindung der Formel I in eine andere Verbindung der Formel I; und
(h) gewünschtenfalls nach den vorstehenden Stufen die Umsetzung einer Verbindung der Formel I mit einer Säure oder einer Base zur Erzielung des entsprechenden Salzes.

10. Pharmazeutische Zusammensetzung, die eine wirksame Menge einer Verbindung der Formel I gemäß einem der Ansprüche 1 bis 8 oder ein pharmazeutisch verträgliches Salz oder Solvat hiervon und einen oder mehrere pharmazeutisch verträgliche Exzipienten umfasst.

11. Verwendung einer Verbindung der Formel I gemäß einem der Ansprüche 1 bis 8 oder eines pharmazeutisch verträglichen Salzes oder Solvats hiervon zur Herstellung eines Arzneimittels für die Behandlung oder Prävention von durch Cyclooxygenase bedingten Erkrankungen.

12. Verwendung einer Verbindung der Formel I gemäß einem der Ansprüche 1 bis 8 oder eines pharmazeutisch verträglichen Salzes oder Solvats hiervon zur Herstellung eines Arzneimittels für die Behandlung oder Prävention von durch Cyclooxygenase-2 bedingten Erkrankungen.

13. Verwendung einer Verbindung der Formel I gemäß einem der Ansprüche 1 bis 8 oder eines pharmazeutisch verträglichen Salzes oder Solvats hiervon zur Herstellung eines Arzneimittels für die Behandlung von Inflammation, Schmerzen und/oder Fieber.

14. Verwendung einer Verbindung der Formel I gemäß einem der Ansprüche 1 bis 8 oder eines pharmazeutisch verträglichen Salzes oder Solvats hiervon zur Herstellung eines Arzneimittels für die Inhibierung der Prostanoid-induzierten Kontraktion der glatten Muskulatur.

15. Verwendung einer Verbindung der Formel I gemäß einem der Ansprüche 1 bis 8 oder eines pharmazeutisch verträglichen Salzes oder Solvats hiervon zur Herstellung eines Arzneimittels für die Behandlung oder Prävention von Dysmenorrhoe, vorzeitigen Wehen, Asthma und Bronchitis.

16. Verwendung einer Verbindung der Formel I gemäß einem der Ansprüche 1 bis 8 oder eines pharmazeutisch verträglichen Salzes oder Solvats hiervon zur Herstellung eines Arzneimittels für die Behandlung oder Prävention von adenomatöser Polyposis familiaris.

17. Verwendung einer Verbindung der Formel I gemäß einem der Ansprüche 1 bis 8 oder eines pharmazeutisch verträglichen Salzes oder Solvats hiervon zur Herstellung eines Arzneimittels für die Behandlung oder Prävention von Krebs.

18. Verwendung gemäß Anspruch 17, worin der Krebs Gastrointestinalkrebs ist.

19. Verwendung gemäß Anspruch 18, worin der Gastrointestinalkrebs Darmkrebs ist.

20. Verwendung einer Verbindung der Formel I gemäß einem der Ansprüche 1 bis 8 oder eines pharmazeutisch verträglichen Salzes oder Solvats hiervon zur Herstellung eines Arzneimittels für die Behandlung oder Prävention von zerebralem Infarkt, Epilepsie und neurodegenerativen Erkrankungen wie der Alzheimer Erkrankung und Demenz.

## Revendications

1. Composé de formule générale I : 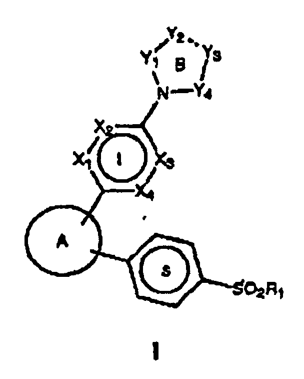 où :
A représente un cycle à 5 chaînons insaturé ou partiellement insaturé, qui peut éventuellement contenir de 1 à 3 hétéroatomes choisis parmi N, O et S, où les deux substituants aromatiques s et t sont placés sur des atomes adjacents du cycle A, et où A peut être éventuellement substitué avec un ou plusieurs substituants R₂ ;
R₁ représente C₁₋₈ alkyle, C₁₋₈ halogénoalkyle ou -NR₃R₄ ;
R₂ représente C₁₋₄ alkyle, C₁₋₄ halogénoalkyle, halogène, oxo, cyano, nitro, -CHO, -COCH₃ ou -COOR₃ ;
R₃ représente l'hydrogène, C₁₋₈ alkyle ou arylC₀₋₈ alkyle ;
R₄ représente l'hydrogène, C₁₋₈ alkyle, arylC₁₋₈ alkyle, -COR₅ ou -COOR₅ ;
R₅ représente C₁₋₈ alkyle ou C₁₋₈ halogénoalkyle ;
X₁, X₂, X₃ et X₄ représentent tous CR₆, ou un, deux ou trois parmi X₁, X₂, X₃ et
X₄ représentent l'azote et les autres groupes X₁, X₂, X₃ et X₄ représentent CR₆ ;
chaque R₆ représente indépendamment l'hydrogène, halogène, C₁₋₃ alkyle ou C₁₋₃ alcoxy ;
les pointillés dans le cycle B représentent une simple ou double liaison ;
Y₁ et Y₄ représentent indépendamment CR₇R₇ ou CO ;
quand les pointillés représentent une double liaison Y₂ et Y₃ représentent CR₈ ;
quand les pointillés représentent une simple liaison Y₂ et Y₃ représentent CR₈R₉, et en outre Y₂ peut représenter CO si Y₁ est différent de CO, et en outre Y₃ peut représenter CO si Y₄ est différent de CO ou Y₃ peut représenter NR₉, O ou S si Y₄ représente CO ;
chaque R₇ représente indépendamment l'hydrogène, méthyle ou éthyle ;
chaque R₈ représente indépendamment l'hydrogène, méthyle, éthyle, hydroxyle, méthoxy ou halogène ;
R₉ représente l'hydrogène ou C₁₋₄ alkyle ;
aryle dans les définitions ci-dessus représente phényle ou naphtyle, qui peut éventuellement être substitué avec un ou plusieurs groupes choisis parmi C₁₋₈ alkyle, halogène, C₁₋₈ halogénoalkyle, cyano, nitro, R₁₀OC₀₋₈ alkyle, R₁₀SC₀₋₈ alkyle, -NR₁₀R₁₁, -NR₁₀COR₁₁, -COR₁₀ ou -COOR₁₀ ;
R₁₀ représente l'hydrogène, C₁₋₈ alkyle ou benzyle ;
R₁₁ représente C₁₋₈ alkyle ou C₁₋₈ halogénoalkyle ;
et ses sels et solvates.

2. Composé selon la revendication 1 où R₁ représente méthyle ou amino.

3. Composé selon la revendication 1 ou 2 où X₁, X₂, X₃ et X₄ représentent tous CR₆, ou un parmi X₂ ou X₃ représente N et les autres représentent CR₆.

4. Composé selon l'une quelconque des revendications 1 à 3 où A représente l'imidazole, le pyrazole, la furanone, le thiophène, l'isoxazole, l'oxazole, l'oxazolone ou le cyclopentène, où A peut éventuellement être substitué avec un ou plusieurs substituants R₂.

5. Composé selon la revendication 4 où A représente l'imidazole, la furanone ou le pyrazole, où A peut éventuellement être substitué avec un ou plusieurs substituants R₂.

6. Composé selon l'une quelconque des revendications 1 à 5 où R₇ et R₈ représentent l'hydrogène.

7. Composé selon l'une quelconque des revendications 1 à 5 où Y₁, Y₂, Y₃ et Y₄ représentent CH₂ et les pointillés représentent une simple liaison ou l'un parmi Y₁ ou Y₄ représente CO et l'autre représente CH₂ et Y₂ et Y₃ représentent CH₂ auquel cas les pointillés représentent une simple liaison ou Y₂ et Y₃ représentent CH auquel cas les pointillés représentent une double liaison.

8. Composé selon la revendication 1 choisi parmi :
le 4-chloro-1-(4-méthylsulfonylphényl)-5-[4-(1-pyrrolidinyl)phényl]imidazole ;
le 1-(4-méthylsulfonylphényl)-5-[4-(1-pyrrolidinyl)phényl]imidazole ;
le 4-chloro-5-[4-(3-hydroxypyrrolidin-1-yl)phényl]-1-(4-méthylsulfonylphényl)-imidazole ;
le 4-chloro-5-[4-(2-méthylpyrrolidin-1-yl)phényl]-1-(4-méthylsulfonylphényl)-imidazole ;
le 4-[4-chloro-5-[4-(1-pyrrolidinyl)phényl]imidazol-1-yl]benzènesulfonamide ;
le 4-chloro-5-[3-chloro-4-(1-pyrrolidinyl)phényl]-1-(4-méthylsulfonylphényl)-imidazole ;
le 4-chloro-1-(4-méthylsulfonylphényl)-5-[4-(2,5-dioxopyrrolidin-1-yl)phényl]-imidazole ;
le 4-chloro-1-(4-méthylsulfonylphényl)-5-[4-(2-oxo-3-pyrrolin-1-yl)phényl]-imidazole ;
le 4-chloro-1-(4-méthylsulfonylphényl)-5-[4-(2-oxooxazolidin-3-yl)phényl]-imidazole ;
le 4-chloro-1-(4-méthylsulfonylphényl)-5-[4-(2-oxopyrrolidin-1-yl)phényl]-imidazole ;
le 1-(4-méthylsulfonylphényl)-5-[6-(1-pyrrolidinyl)-3-pyridyl]imidazole ;
le 4-chloro-1-(4-méthylsulfonylphényl)-5-[6-(1-pyrrolidinyl)-3-pyridyl]imidazole ;
la 3-[4-(2,5-dioxopyrrolidin-1-yl)phényl]-4-(4-méthylsulfonylphényl)-*5H*-furan-2-one ;
la 4-(4-méthylsulfonylphényl)-3-[4-(2-oxo-3-pyrrolin-1-yl)phényl]-*5H*-furan-2-one ;
la 4-(4-méthylsulfonylphényl)-3-[4-(1-pyrrolidinyl)phényl]-*5H*-furan-2-one ;
la 3-[3-chloro-4-(1-pyrrolidinyl)phényl]-4-(4-méthylsulfonylphényl)-*5H*-furan-2-one ;
la 4-(4-méthylsulfonylphényl)-3-[6-(1-pyrrolidinyl)-3-pyridyl]-*5H*-furan-2-one ;
le 4-[5-[4-(2-oxo-3-pyrrolin-1-yl)phényl]-3-trifluorométhyl-*1H*-pyrazol-1-yl]-benzènesulfonamide ;
le 4-[5-[4-(1-pyrrolidinyl)phényl]-3-trifluorométhyl-*1H*-pyrazol-1-yl]benzènesulfonamide ;
ou sel ou solvate de celui-ci.

9. Procédé pour préparer un composé de formule I selon la revendication 1 qui comprend :
(a) quand, dans un composé de formule I, Y₁ et Y₄ représentent CR₇R₇, la réaction d'un composé de formule II 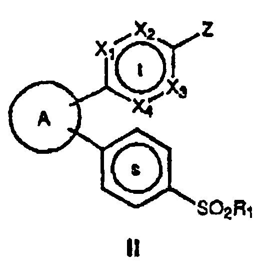 où X₁, X₂, X₃, X₄, R₁ et A ont la signification décrite dans la revendication 1 et Z représente fluoro ou, quand l'un parmi X₂ ou X₃ représente l'azote, Z représente chloro, avec une amine de formule IV 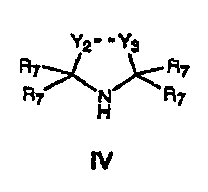 où R₇, Y₂, Y₃ et les pointillés ont la signification décrite dans la revendication 1 ; ou
(b) quand, dans un composé de formule I, Y₁ et Y₄ représentent CR₇R₇, la réaction d'un composé de formule III 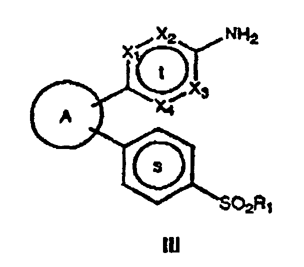 où X₁, X₂, X₃, X₄, R₁ et A ont la signification décrite dans la revendication 1, avec un composé de formule V 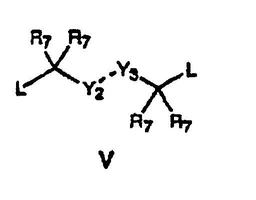 où R₇, Y₂, Y₃ et les pointillés ont la signification décrite dans la revendication 1 et L représente un bon groupe partant ; ou
(c) quand, dans un composé de formule I, Y₁ et/ou Y₄ représentent CO, la réaction d'un composé de formule III avec un composé de formule VI ou VI' respectivement 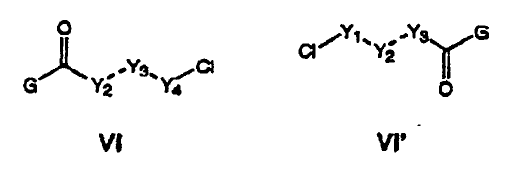 où Y₁, Y₂, Y₃, Y₄ et les pointillés ont la signification décrite dans la revendication 1 et G représente un bon groupe partant ; ou
(d) quand, dans un composé de formule I, Y₄ représente CO et Y₃ représente NH, la réaction d'un composé de formule III avec un isocyanate de formule VII 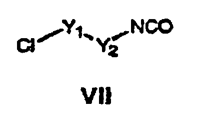 où Y₁ et Y₂ ont la signification décrite dans la revendication 1 ; ou
(e) quand, dans un composé de formule I, Y₁ et Y₄ représentent CO, la réaction d'un composé de formule III avec un composé de formule VIII 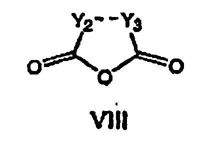 où Y₂, Y₃ et les pointillés ont la signification décrite dans la revendication 1 ; ou
(f) quand, dans un composé de formule I, l'un parmi Y₁ ou Y₄ représente CH₂ et l'autre représente CO et les pointillés représentent une double liaison, la réaction d'un composé de formule III avec un composé de formule IX 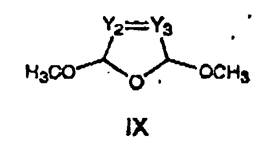 où Y₂ et Y₃ ont la signification décrite dans la revendication 1 ; ou
(g) la conversion, en une étape ou une pluralité d'étapes, d'un composé de formule I en un autre composé de formule I ; et
(h) si on le souhaite, après les étapes ci-dessus, la réaction d'un composé de formule I avec un acide ou une base pour donner le sel correspondant.

10. Composition pharmaceutique qui comprend une quantité efficace d'un composé de formule I selon l'une quelconque des revendications 1 à 8 ou d'un sel ou solvate pharmaceutiquement acceptable de celui-ci et un ou plusieurs excipients pharmaceutiquement acceptables.

11. Utilisation d'un composé de formule I selon l'une quelconque des revendications 1 à 8 ou d'un sel ou solvate pharmaceutiquement acceptable de celui-ci pour la fabrication d'un médicament pour le traitement ou la prévention de maladies à médiation par une cyclooxygénase.

12. Utilisation d'un composé de formule I selon l'une quelconque des revendications 1 à 8 ou d'un sel ou solvate pharmaceutiquement acceptable de celui-ci pour la fabrication d'un médicament pour le traitement ou la prévention de maladies à médiation par la cyclooxygénase-2.

13. Utilisation d'un composé de formule I selon l'une quelconque des revendications 1 à 8 ou d'un sel ou solvate pharmaceutiquement acceptable de celui-ci pour la fabrication d'un médicament pour le traitement de l'inflammation, de la douleur et/ou de la fièvre.

14. Utilisation d'un composé de formule I selon l'une quelconque des revendications 1 à 8 ou d'un sel ou solvate pharmaceutiquement acceptable de celui-ci pour la fabrication d'un médicament pour inhiber la contraction des muscles lisses induite par des prostanoïdes.

15. Utilisation d'un composé de formule I selon l'une quelconque des revendications 1 à 8 ou d'un sel ou solvate pharmaceutiquement acceptable de celui-ci pour la fabrication d'un médicament pour le traitement ou la prévention de la dysménorrhée, du travail avant terme, de l'asthme et de la bronchite.

16. Utilisation d'un composé de formule I selon l'une quelconque des revendications 1 à 8 ou d'un sel ou solvate pharmaceutiquement acceptable de celui-ci pour la fabrication d'un médicament pour le traitement ou la prévention de la polypose adénomateuse familiale.

17. Utilisation d'un composé de formule I selon l'une quelconque des revendications 1 à 8 ou d'un sel ou solvate pharmaceutiquement acceptable de celui-ci pour la fabrication d'un médicament pour le traitement ou la prévention du cancer.

18. Utilisation selon la revendication 17 où le cancer est un cancer gastro-intestinal.

19. Utilisation selon la revendication 18 où le cancer gastro-intestinal est le cancer du côlon.

20. Utilisation d'un composé de formule I selon l'une quelconque des revendications 1 à 8 ou d'un sel ou solvate pharmaceutiquement acceptable de celui-ci pour la fabrication d'un médicament pour le traitement ou la prévention de l'infarctus cérébral, de l'épilepsie, et de maladies neurodégénératives comme la maladie d'Alzheimer et la démence.
